# EUROPÄISCHE PATENTSCHRIFT

(11) **EP 3 206 652 B1**
(45) Veröffentlichungstag und Bekanntmachung des Hinweises auf die Patenterteilung: **15.02.2023**
(21) Anmeldenummer: 15781871.7
(22) Anmeldetag: 19.10.2015
(51) Int. Cl.: A61G 12/00, F16M 13/02

(54) **MONTAGEVORRICHTUNG FÜR EINE STATIVVORRICHTUNG SOWIE MONTAGESYSTEM MIT DER MONTAGEVORRICHTUNG**
ASSEMBLY DEVICE FOR A STAND DEVICE AND ASSEMBLY SYSTEM WITH ASSEMBLY DEVICE
DISPOSITIF DE MONTAGE POUR UN DISPOSITIF À PIED ET SYSTÈME DE MONTAGE DOTÉ D'UN DISPOSITIF DE MONTAGE

(30) Priorität: 17.10.2014 EP 14003555
(43) Veröffentlichungstag der Anmeldung: 23.08.2017
(73) Patentinhaber: Ondal Medical Systems GmbH, 36088 Hünfeld (DE)
(72) Erfinder: OGINSKI, Stefan, 36041 Fulda (DE); HÖSER, Markus, 36142 Tann (DE)
(74) Vertreter: Graf von Stosch, Andreas
(86) Internationale Anmeldenummer: PCT/EP2015/002061
(87) Internationale Veröffentlichungsnummer: WO 2016/058706

(56) Entgegenhaltungen:
- WO-A1-94/27549
- GB-A- 2 489 608
- US-A- 3 814 023
- US-A- 4 840 278
- US-A- 4 901 967

## Beschreibung

Die vorliegende Erfindung betrifft nach dem Oberbegriff des Anspruchs 1 eine Montagevorrichtung zur Montage einer Stativvorrichtung unter einer Raumdecke im Operationssaal, umfassend: eine sich in einer Längsrichtung entlang einer Montageachse erstreckende Montageeinrichtung eingerichtet zum Halten eines Verbindungsbauteils der Stativvorrichtung, insbesondere einer Spindel; und einen Deckenflansch eingerichtet zum Montieren der Montageeinrichtung unter der Raumdecke und zum Lagern der Stativvorrichtung an der Raumdecke, wobei der Deckenflansch in unterschiedlichen Montagepositionen entlang der Montageachse (D) relativ zur Montageeinrichtung an der Montageeinrichtung derart montierbar ist, dass die Montagevorrichtung eingerichtet ist, die Stativvorrichtung höhenvariabel in unterschiedlichen, insbesondere vordefinierten Höhenpositionen zu halten. Die vorliegende Erfindung betrifft auch ein Montagssystem mit einer derartigen Montagevorrichtung sowie eine Stativvorrichtung mit einem derartigen Montagesystem.

Stative, insbesondere Deckenstative wie z.B. Deckenversorgungseinheiten, Monitorträger, oder so genannte Federarme oder Zentralachsen, weisen meist einen oder mehrere in Bezug auf eine Vertikalposition starr angeordneten oder höhenverstellbaren Träger auf, mittels welchen eine daran befestigte medizintechnische Einrichtung bewegt und positioniert werden kann, z.B. im Operationssaal, insbesondere auch auf einer Intensivstation. An den Stativen sind häufig Versorgungseinheiten montiert, an welchen z.B. medizinisch-elektrische Endgeräte angeordnet sind, die z.B. während einer Operation mit den benötigten Medien versorgt werden. Die Träger definieren dabei einen Aktionsradius der medizintechnischen Einrichtung, in welchem die medizintechnische Einrichtung positioniert werden kann. Die Träger können meist zumindest um mindestens eine drehbare Verbindung, insbesondere ein Drehgelenk verdreht werden. Wahlweise sind die Träger auch höhenverstellbar und/oder um eine zumindest annähernd horizontal ausgerichtete Achse in der Höhe verschwenkbar angeordnet.

Die Montage des Stativs erfolgt häufig an der Raumdecke bzw. angrenzend an eine Zwischendecke des Operationssaals. Die Zwischendecke selbst dient dabei zum Lagern von z.B. Kabeln, nicht aber zum Abstützen des Stativs. Hierzu weist das Stativ z.B. ein Deckenrohr auf. Dabei muss häufig eine Anpassung der Höhenposition des Stativs relativ zur Raumdecke erfolgen. Über die Höhenposition des Stativs relativ zu einem Montagepunkt an der Raumdecke kann die Position einer vom Stativ gehaltenen medizintechnischen Einrichtung vorgegeben werden. Häufig ist auch die exakte Höhenposition der Zwischendecke nicht bekannt. Das Stativ hingegen soll möglichst in einer exakt vorgegebenen Höhe montiert werden können.

Zur Montage des Stativs können z.B. Montagevorrichtungen mit auf eine Spindel des Stativs aufgeschrumpften Scheiben verwendet werden. Die aufgeschrumpften Scheiben können mit dem Deckenrohr verschraubt sein.

Für eine Höhenanpassung können höhenverstellbare Deckenrohre verwendet werden. Dabei kann z.B. eine Klemmverschraubung mit ringförmigen Keilsegmenten durch Kraftschluss eine variierbare Höhenposition sicherstellen. Eine zusätzlich erforderliche formschlüssige Bolzensicherung kann dabei eine relative Position des Deckenrohres an einem Deckenflansch sicherstellen. Die für den Bolzen in einer bestimmten Position erforderliche Bohrung muss dann jeweils im Einzelfall an der richtigen Position nachträglich bei der Montage eingebracht werden. Das Deckenrohr muss dabei möglicherweise höher als der Deckenflansch geschoben werden. Gegebenenfalls muss das Deckenrohr gekürzt werden, wenn oberhalb des Deckenflansch nicht genügend Platz zur Verfügung steht.

Auch sind Vorrichtungen bekannt, bei welchen ein Deckenflansch, an welchem die Spindel fest vormontiert ist, mittels Gewindestangen und wahlweise auch Distanzrohren an eine so bezeichnete Schnittstellenplatte montiert wird, welche wiederum an eine so bezeichnete Deckenplatte montiert wird, ebenfalls mittels Gewindestangen und Distanzrohren. Mittels der Gewindestangen ist eine Höhenanpassung und auch Ausrichtung möglich.

WO 94/27549 A1 beschreibt ein Zwischengestell für die Befestigung eines Deckenstativs an einer Decke mit einem Deckenanl<erteil, einschließlich einer Mehrzahl von mit diesem verbundenen Distanzstücken und einer Schnittstellenplatte. Das Deckenankerteil besteht aus einer Mehrzahl von miteinander verbindbaren Stücken, und einer Mehrzahl von von den Stücken getragenen Distanzstücken, die mit ihren Enden an den Stücken befestigt sind.

US 3,814,023 A offenbart ein beispielsweise an einer Decke montierbares Trägersystem, das teleskopartige Röhren umfasst. Arme können höhenvariabel verstellt werden.

Es ist eine Aufgabe der vorliegenden Erfindung, eine Montagevorrichtung bereitzustellen, mittels welcher die Stativvorrichtung in einer bestimmten Höhenposition an einer Raumdecke montiert werden kann, ohne dass aufwändige konstruktive Änderungen oder Anpassungen der Montagevorrichtung erforderlich sind. Die Aufgabe besteht insbesondere auch darin, eine Montagevorrichtung bereitzustellen, mittels welcher ein Einstellen oder Nachjustieren der Höhenposition auf einfache Weise erfolgen kann, insbesondere auch auf manuelle Weise. Die Montagevorrichtung soll sich dabei bevorzugt auch durch eine hohe Tragfähigkeit auszeichnen und zur Aufnahme und Weiterleitung von hohen Gewichtskräften geeignet sein. Bevorzugt weist die Montagevorrichtung dabei einen einfachen konstruktiven Aufbau auf und ist kostengünstig herstellbar.

Diese Aufgabe wird gelöst durch eine Montagevorrichtung zur Montage einer Stativvorrichtung unter einer Raumdecke im Operationssaal nach Anspruch 1, umfassend: eine sich in einer Längsrichtung entlang einer Montageachse erstreckende Montageeinrichtung eingerichtet zum Halten eines Verbindungsbauteils der Stativvorrichtung, insbesondere einer Spindel; und einen Deckenflansch eingerichtet zum Montieren der Montageeinrichtung unter der Raumdecke und zum Lagern der Stativvorrichtung an der Raumdecke; wobei der Deckenflansch in unterschiedlichen Montage-Positionen entlang der Montageachse relativ zur Montageeinrichtung an der Montageeinrichtung derart montierbar ist, dass die Montagevorrichtung eingerichtet ist, die Stativvorrichtung höhenvariabel in unterschiedlichen insbesondere vordefinierten Höhenpositionen zu halten. Die Montagevorrichtung weist einen Deckenflansch und eine Montageeinrichtung auf, welche derart konstruktiv aufeinander abgestimmt sind, dass der Deckenflansch und die Montageeinrichtung in unterschiedlichen Kupplungspunkten/Befestigungspunkten in unterschiedlichen Relativpositionen aneinander kuppelbar sind. Hierdurch kann eine Höhenposition je nach Raumsituation oder Größe der Stativvorrichtung definiert werden.

Der Deckenflansch kann in mindestens zwei vordefinierten Höhenpositionen an der Montageeinrichtung montierbar sein. Die Montagevorrichtung kann dabei in einer gut zugänglichen Position, z.B. am Boden eingestellt und zusammengeschraubt werden und dann an der Decke montiert werden. Dabei ist es zweckmäßig, vorher die Höhe der Raumdecke bzw. der Zwischendecke zu messen, um die geeignete Höhenposition einstellen zu können.

Die Längsrichtung entspricht dabei in einem montierten Zustand einer Höhenrichtung, insbesondere einer vertikalen Richtung.

Als Montageeinrichtung ist dabei eine Einrichtung zu verstehen, mittels welcher die Stativvorrichtung an einer Raumdecke montierbar ist und mittels welcher eine von der Stativvorrichtung ausgeübte Gewichtskraft oder Trägheitskräfte auf die Raumdecke übertragen werden kann, und zwar mittels eines Deckenflansches. Die Montageeinrichtung wird mittels mindestens zweier Halter an einem Deckenflansch montiert.

Die Montageeinrichtung weist mindestens zwei Befestigungsabschnitte mit einer Mehrzahl von insbesondere identisch ausgebildeten Befestigungsmitteln auf, welche in Längsrichtung beabstandet zueinander angeordnet sind. Die Befestigungsmittel ermöglichen eine Mehrzahl von unterschiedlichen relativen Positionen der Montageeinrichtung relativ zum Deckenflansch. Bevorzugt sind die Befestigungsmittel in einer Richtung quer bzw. orthogonal zur Längsrichtung ausgerichtet.

Die Montagevorrichtung umfasst mindestens zwei Halter, mittels welcher die Montageeinrichtung in unterschiedlichen Relativpositionen an den Deckenflansch kuppelbar ist, wobei jeder Halter jeweils einen Befestigungsabschnitt aufweist, welcher geometrisch korrespondierend zum Befestigungsabschnitt der Montageeinrichtung ausgebildet ist. Dabei ist der Halter eingerichtet zum Verbinden des Deckenflansch mit der Montageeinrichtung und zum Übertragen einer von der Stativvorrichtung ausgeübten Last, insbesondere Gewichtskraft, von der Montageeinrichtung auf den Deckenflansch.

Gemäß der vorliegenden Erfindung umfasst die Montagevorrichtung eine Montageeinrichtung, die rohrförmig ausgebildet ist, wobei die Befestigungsabschnitte als radial von der Aussenkontur der Montageeinrichtung abragende Stege ausgebildet sind, wobei weiterhin wenigstens zwei, bevorzugt mindestens drei Befestigungsabschitte an der Montageeinrichtung vorgesehen sind, die mit einer korrespondierenden Anzahl an Haltern am Deckenflansch zusammenwirken. Die zur Verbindung von Deckenflansch und Montageeinrichtung mit der darin gehaltenen Spindel notwendigen Befestigunselemente durchsetzten bzw. penetrieren also nicht den rohrförmigen Körper der Montageeinrichtung sondern nur die radial aussen angeordneten Stege, so dass der rohrförmige Teil der Montageeinrichtung nicht geschwächt wird. Auf diese Weise kann eine extrem robuste und hohe Zugkräfte aufnehmende Montagevorrichtung bereitgestellt werden.

So ist die Montageeinrichtung rohrartig ausgebildet, wobei mindestens zwei oder drei Befestigungsabschnitte in Form von Stegen an einer Außenkontur der Montageeinrichtung angeordnet sind, bevorzugt in demselben Abstand in Umfangsrichtung zueinander. Dies ermöglicht eine stabile Befestigung jeweils an einem in Hinblick auf Drehmomente vorteilhaften (weit außen liegenden) Befestigungspunkt, oder auch mit besonders symmetrischer Kraftverteilung. Bevorzugt sind drei Befestigungsabschnitte vorgesehen, welche jeweils in einem Abstand entsprechend einem Umfangswinkel von 120° zueinander an der Außenkontur angeordnet sind.

Die mindestens zwei Befestigungsabschnitte sind an einem in radialer Richtung an einer Außenseite oder Außenkontur der Montageeinrichtung abstehenden Steg angeordnet oder bildet diesen Steg. Diese Anordnung ermöglicht z.B. auch eine beidseitige Zugänglichkeit des Befestigungsabschnitts. Der Steg kann auch als Lasche beschrieben werden oder als Lasche ausgebildet sein.

Weil die mindestens zwei Befestigungsabschnitte an einer Außenseite, insbesondere Außenkontur oder Außenmantelfläche der Montageeinrichtung angeordnet sind, kann eine gute Zugänglichkeit sichergestellt werden. Die Befestigungsmittel der Montageeinrichtung sind in Längsrichtung bevorzugt in regelmäßigen Abständen zueinander angeordnet. Bevorzugt ist ein jeweiliger Befestigungsabschnitt in Form einer Lochleiste ausgebildet.

Als Stativvorrichtung ist dabei bevorzugt eine Vorrichtung zum Halten, ortsfesten Anordnen und/oder Verlagern mindestens einer medizintechnische Einrichtung zu verstehen, die an einer Wand (in einem Wandlager) oder einer Raumdecke oder auch am Boden eines Operationssaals oder irgendeines anderen Raumes für medizinische Zwecke fest montiert oder positioniert werden kann, also z.B. ein Deckenstativ. Die Stativvorrichtung ist dann nicht vollkommen frei im Operationssaal verlagerbar, sondern kann nur in einem bestimmten Aktionsradius verlagert werden, insbesondere relativ zu einem an einer Raumdecke oder Wand des Operationssaals angeordneten Befestigungspunkt bzw. Montagepunkt. Die Stativvorrichtung kann als an einer Raumdecke montierte Deckenversorgungseinheit ausgebildet sein und eine oder mehrere Versorgungskonsolen aufweisen, welche an einem oder zwei Tragarmen gelagert und positionierbar ist. Die Stativvorrichtung kann auch als Monitorträger ausgebildet sein. Die Stativvorrichtung kann auch als so genannter, insbesondere an einer Wand montierter Federarm ausgebildet sein und z.B. eine Leuchte aufweisen. Die Stativvorrichtung kann auch als so genannte, insbesondere an einer Raumdecke montierte Zentralachse ausgebildet sein und eine Mehrzahl von Tragsystemen mit jeweils mindestens einem Träger aufweisen, an welchem z.B. ein Monitor oder eine Leuchte gelagert ist. Bevorzugt weist die Stativvorrichtung mindestens zwei Tragarme auf.

Als medizintechnische Einrichtung ist dabei bevorzugt eine Versorgungskonsole zu verstehen, mittels welcher Mittel für eine Versorgung eines Patienten und/oder Instrumente für einen Operateur und/oder Licht, Reinluft oder andere im Operationssaal benötigte Medien bereitgestellt werden können. Die medizintechnische Einrichtung weist bevorzugt irgendein Bedienpanel und/oder irgendeine Anzeigevorrichtung zum grafischen Darstellen von z.B. Patientendaten auf.

Als Verbindungsbauteil ist dabei bevorzugt ein Bauteil zu verstehen, mittels welches einzelne Träger der Stativvorrichtung mit der Montagevorrichtung und bevorzugt auch untereinander verbunden werden können. Bei einer Stativvorrichtung in Form einer so genannten Zentralachse kann das Verbindungsbauteil als zentral angeordnete Spindel ausgebildet sein, an welcher mehrere Träger oder Tragarme gelagert sind.

Als Operationssaal ist dabei auch ein Untersuchungsraum oder eine Intensivstation aufzufassen, also ein Raum zum Durchführen medizinischer Behandlungen oder Therapien.

Gemäß einem Ausführungsbeispiel erstrecken sich die mindestens zwei Befestigungsabschnitte in Längsrichtung jeweils entlang der gesamten Länge der Montageeinrichtung. Dies ermöglicht eine Höhenanpassung mit einer besonders großen Flexibilität. Die gesamte Länge der Montageeinrichtung kann genutzt werden, um einzelne Höhenpositionen einzustellen.

Bevorzugt weist ein Befestigungsabschnitt der Montageeinrichtung jeweils mindestens eine, bevorzugt zwei radial ausgerichtete ebene Radialflanken auf (insbesondere gegenüberliegende Seitenflächen), welche bevorzugt entlang des gesamten Befestigungsabschnitts vorgesehen sind. Der Deckenflansch oder ein Halter kann wahlweise an einer oder mehrerer dieser Radialflanken angeordnet werden und daran in Längsrichtung verschoben werden. Wahlweise kann der Deckenflansch oder ein Halter auch zwei gegenüberliegende Radialflanken des jeweiligen Befestigungsabschnitts der Montageeinrichtung umgreifen. Die Montageeinrichtung kann mittels der Radialflanken dabei auch exakt in einer vorbestimmten Position relativ zum Deckenflansch positioniert werden.

Gemäß einem Ausführungsbeispiel weist der Deckenflansch eine Durchführung auf, welche bevorzugt einen Innendurchmesser hat, der größer oder größer gleich ist als ein Außendurchmesser der Montageeinrichtung. Dies ermöglicht ein Durchstecken/Hindurchführen der Montageeinrichtung durch den Deckenflansch. Hierdurch können eine Vielzahl von relativen Höhenpositionen/Montagepositionen bereitgestellt werden. Die Montageeinrichtung braucht dabei selbst dann nicht gekürzt werden, wenn der Deckenflansch verhältnismäßig weit unten befestigt werden soll, insbesondere an einem unteren Ende der Montageeinrichtung, um eine besonders hohe Position der Stativvorrichtung zu ermöglichen. Somit kann auf einfache Weise, insbesondere ohne Änderung der Komponenten, auch eine verhältnismäßig hohe Höhenposition der Stativvorrichtung eingestellt werden.

Die Durchführung weist bevorzugt eine Innenkontur auf, die geometrisch korrespondierend zu einer Außenkontur der Montageeinrichtung ausgebildet ist. Hierdurch kann z.B. auch eine formschlüssige Verdrehsicherung mittels des Deckenflansch bereitgestellt werden. Bevorzugt weist die Durchführung in radialer Richtung ausgerichtete Aussparungen auf, welche geometrisch korrespondierend zu Stegen der Montageeinrichtung ausgebildet sind. Die Aussparungen können die Positionierung und Führung der Montageeinrichtung erleichtern, insbesondere auch bei der Montage von winkelförmigen, separaten Haltern am Deckenflansch und an der Montageeinrichtung.

Die Innenkontur bildet bevorzugt mindestens eine Radialnut, welche geometrisch korrespondierend zu einem Befestigungsabschnitt, insbesondere Steg der Montageeinrichtung ausgebildet ist. Hierdurch kann die Montageeinrichtung ohne spürbares Verdrehspiel (unbeachtlich eines durch Fertigungstoleranzen bedingten Verdrehspiels) am Deckenflansch gelagert werden.

Gemäß einem Ausführungsbeispiel weist der Deckenflansch eine ebene unterseitige Stirnfläche auf, an welcher die Montageeinrichtung oder ein Halter zur Anlage bringbar ist, wobei der Deckenflansch bevorzugt als Flanschplatte ausgebildet ist. Hierdurch kann eine Kontaktfläche zur Kraftübertragung und zur vordefinierbaren Positionierung bereitgestellt werden.

Gemäß einem Ausführungsbeispiel weist der Deckenflansch eine Mehrzahl von Befestigungsmitteln, insbesondere Innengewindebohrungen oder Durchgangslöcher auf, welche zur Befestigung der Montageeinrichtung am Deckenflansch eingerichtet sind, und welche bevorzugt in Längsrichtung ausgerichtet sind. Dies erleichtert die Montage. Die Befestigungsmittel oder daran angreifende Befestigungselemente können dabei in eine vorherrschende Kraftflussrichtung ausgerichtet sein. Bevorzugt sind die Befestigungsmittel als Durchgangsbohrungen ausgebildet. Durchgangslöcher ermöglichen, Schrauben von oben durch zu stecken.

Gemäß einem Ausführungsbeispiel ist der Halter als separates Teil separat vom Deckenflansch und separat von der Montageeinrichtung vorgesehen. Wahlweise kann der Halter am Deckenflansch vormontiert sein oder einstückig durch einen in axialer Längsrichtung hervorstehenden Abschnitt des Deckenflansch gebildet sein. Gemäß einer Variante sind Halter und Deckenflansch als Gussteil ausgebildet.

Gemäß einem Ausführungsbeispiel weist der Halter mindestens ein Befestigungsmittel auf, welches geometrisch korrespondierend zu den Befestigungsmitteln der Montageeinrichtung angeordnet und/oder ausgebildet ist. Hierdurch können der Halter und die Montageeinrichtung in einer vordefinierbaren Anordnung relativ zueinander positioniert werden. An den Befestigungsmitteln können Befestigungselemente, insbesondere Schrauben montiert werden. Gemäß einer Variante kann die Montageeinrichtung mittels der mindestens zwei Halter dabei auch ohne Befestigungselemente formschlüssig und/oder kraftschlüssig gehalten werden. Dies kann insbesondere dadurch sichergestellt werden, dass der Halter elastisch vorgespannt an der Montageeinrichtung angreifen kann und die Montageeinrichtung (dessen Eigengewicht) halten kann. Eine zusätzliche Bolzensicherung ist nicht erforderlich. Dies liefert bei der Montage den Vorteil, dass zunächst der Halter am Deckenflansch montiert werden kann, um daraufhin die Montageeinrichtung am Halter in einer gewünschten Höhenposition so zu positionieren, dass die jeweiligen Befestigungsmittel zueinander fluchtend angeordnet sind. Befestigungselemente können dann angebracht/montiert werden, ohne dass die Montageeinrichtung festgehalten werden muss.

Die mindestens zwei Befestigungsabschnitte der Montageeinrichtung weisen in Längsrichtung bevorzugt eine Erstreckung größer als der jeweilige Halter auf. Dies ermöglicht ein axiales Verschieben des Halters entlang des Befestigungsabschnitts. Bevorzugt weisen die Halter in der Längsrichtung eine Erstreckung auf, die mehr als doppelt so groß ist wie der Abstand von zwei in Längsrichtung nebeneinander angeordneten Befestigungsmitteln der Montageeinrichtung zueinander. Hierdurch kann sichergestellt werden, dass die Montageeinrichtung in unterschiedlichen Abständen relativ zum Deckenflansch positioniert werden kann, ohne dass die Montageeinrichtung gekürzt werden muss oder durch den Deckenflansch hindurchgeführt werden muss. Nichtsdestotrotz kann die Montageeinrichtung auch durch den Deckenflansch hindurchgeführt werden und oben aus dem Deckenflansch herausragen. Bevorzugt weisen die Halter in der Längsrichtung eine Erstreckung auf, die mehr als dreimal so groß ist wie der Abstand von zwei Befestigungsmitteln der Montageeinrichtung zueinander. Hierdurch können mehrere Höhenpositionen eingestellt werden.

Gemäß einer Variante ist der Halter ein L-förmiger Winkel und weist eine ebene Längsseite sowie eine ebene Stirnseite auf, mittels welcher der Halter jeweils am Deckenflansch und an der Montageeinrichtung zur Anlage bringbar ist.

Gemäß einem Ausführungsbeispiel weist der Halter eine Kavität auf, insbesondere eine in radialer Richtung ausgerichtete Radialkavität, welche geometrisch derart korrespondierend zum Befestigungsabschnitt der Montageeinrichtung ausgebildet ist, dass der Halter eingerichtet ist, den Befestigungsabschnitt in radialer Richtung zu umgreifen. Hierdurch kann die Montageeinrichtung im Halter in Längsrichtung geführt werden und vom Halter oder mehreren Haltern zentriert werden.

Gemäß einer Variante weist der Halter zwei gegenüberliegende Backen auf, welche jeweils eine die Kavität in Umfangsrichtung begrenzende ebene Radialflanke (Seitenfläche) bilden, an welcher der Befestigungsabschnitt der Montageeinrichtung anliegt. Dabei kann der Halter auch als Winkel ausgebildet sein.

Bevorzugt weist der Halter eine die Kavität in radialer Richtung begrenzende Zentrierfläche auf, welche geometrisch korrespondierend zu einem radial außenliegenden Umfangsflächenabschnitt des Befestigungsabschnitts ausgebildet ist. Dies ermöglicht eine Zentrierung der Montageeinrichtung mittels des Halters oder mehrerer Halter relativ zum Deckenflansch.

Bevorzugt weist der Halter eine Wellung auf, welche derart angeordnet ist, dass der Halter zwecks erleichterter Montage an der Montageeinrichtung an der Wellung verformbar ist.

Gemäß einem Ausführungsbeispiel ist die Montageeinrichtung ein rohrartiges Stranggussteil, insbesondere aus Aluminium, wobei die Montageeinrichtung bevorzugt einstückig ist. Der Begriff Aluminium kann dabei auch Aluminiumlegierungen umfassen. Bevorzugt wird die Aluminiumlegierung AlMgSi_{0.5} verwendet. Diese Ausgestaltung ermöglicht zum einen eine hohe Stabilität, zum anderen auch eine kostengünstige Herstellungsweise. Dabei kann die axiale Erstreckung um die Montageachse weitgehend frei gewählt werden, je nach Einsatzbedingungen und Montageposition. Bevorzugt ist auch ein Halter zum Verbinden der Montageeinrichtung mit einem Deckenflansch als Stranggussteil ausgebildet, insbesondere aus Aluminium.

Nicht beansprucht ist eine Variante, bei der keine Halter vorgesehen sind. Die Montageeinrichtung weist dabei bspw. Schraubkanäle auf, insbesondere am Rand, mittels welcher die Montageeinrichtung direkt an der/einer Flanschplatte montierbar ist. Hierdurch kann nicht zuletzt eine besonders kostengünstige Variante bereitgestellt werden. Die Anzahl von Komponenten oder Teilen ist reduziert. Die Montage kann weiter vereinfacht werden, insbesondere bei solchen Anwendungen, welche keine besonders große Flexibilität in Hinblick auf Höhenverstellung erfordern.

Gemäß einem Ausführungsbeispiel weist die Montageeinrichtung mindestens zwei Befestigungsabschnitte mit einer Mehrzahl von Befestigungsmitteln aufweist, welche in Längsrichtung beabstandet zueinander angeordnet sind, wobei die mindestens zwei Befestigungsabschnitte an einer Außenseite der Montageeinrichtung angeordnet ist und als in radialer Richtung abstehender Steg ausgebildet ist, wobei die mindestens zwei Befestigungsabschnitte sich in Längsrichtung jeweils entlang der gesamten Länge der Montageeinrichtung erstrecken, wobei die Montagevorrichtung mindestens zwei Halter umfasst, mittels welcher die Montageeinrichtung in unterschiedlichen Relativpositionen an den Deckenflansch kuppelbar ist, wobei jeder Halter einen Befestigungsabschnitt aufweist, welcher geometrisch korrespondierend zum Befestigungsabschnitt der Montageeinrichtung ausgebildet ist, wobei jeder Halter mindestens ein Befestigungsmittel aufweist, welches geometrisch korrespondierend zu Befestigungsmitteln der Montageeinrichtung angeordnet und/oder ausgebildet ist, wobei der Deckenflansch eine unterseitige Stirnfläche aufweist, an welcher die Halter zur Anlage bringbar sind, wobei der Deckenflansch eine Mehrzahl von Befestigungsmitteln aufweist, welche in Längsrichtung ausgerichtet sind und zur Montage der Halter am Deckenflansch eingerichtet sind. Durch diese Ausgestaltung lassen sich eine Vielzahl der mit der vorliegenden Erfindung erzielbaren Vorteile verwirklichen.

Gemäß einem Ausführungsbeispiel weist die Montagevorrichtung zwei unterschiedlich groβe Passflächen auf, welche korrespondierend zu Passflächen des Verbindungsbauteils ausgebildet sind und eingerichtet ist zur Montagevereinfachung und Zweipunktlagerung des Verbindungsbauteils. Dies hat Vorteile in Hinblick auf eine sichere Montage und eine belastbare Lagerung.

Die zuvor genannte Aufgabe wird auch gelöst durch ein Montagesystem mit einer erfindungsgemäßen Montagevorrichtung, wobei das Montagesystem ferner umfasst: mindestens ein Befestigungselement, insbesondere eine Schraube, zur Montage des Deckenflansch an der Montageeinrichtung oder an einem mit der Montageeinrichtung verbundenen Halter, und/oder mindestens ein Befestigungselement, insbesondere eine Schraube, zur Montage mindestens eines Halters an der Montageeinrichtung. Ein solches System liefert einen großen Grad an Flexibilität bei der Montage oder bei der Auswahl einer bestimmten Höhenposition.

Gemäß einem Ausführungsbeispiel umfasst das Montagesystem ferner eine Justageeinrichtung zur Anordnung eines Verbindungsbauteils der Stativvorrichtung in einer vordefinierbaren Position relativ zur Montageeinrichtung, wobei die Montagevorrichtung eine um die Montageachse einstellbare Drehkupplung zum Lagern des Verbindungsbauteils an der Montageeinrichtung bildet. Hierdurch kann zusätzlich zur einstellbaren Höhenposition auch ein Einstellen oder Nachjustieren der relativen Drehposition der Spindel relativ zur Montagevorrichtung auf einfache Weise erfolgen, wie im Folgenden näher erläutert.

Eine Stativvorrichtung nach der vorliegenden Erfindung mit einem erfindungsgemäßen Montagesystem weist ein Verbindungsbauteil, bevorzugt in Form einer Spindel auf, welche axialfest an der Montageeinrichtung gelagert oder befestigt ist. Bevorzugt ist die Spindel verdrehbar an der Montageeinrichtung gelagert. Im Folgenden werden in Hinblick auf eine verdrehbar gelagerte Spindel weitere Varianten oder Merkmale beschrieben, welche auf vorteilhafte Weise mit der erfindungsgemäßen Montagevorrichtung realisiert werden können.

Gemäß einer Variante ist die Montagevorrichtung zum örtlichen Verlagern einer medizintechnischen Einrichtung im Operationssaal eingerichtet, wobei die Montageeinrichtung eine in Längsrichtung ausgerichtete Kavität zur Aufnahme eines drehbar lagerbaren Verbindungsbauteils der Stativvorrichtung aufweist, und wobei die Montagevorrichtung ferner umfasst: eine Justageeinrichtung zur Anordnung des Verbindungsbauteils in einer vordefinierbaren Position relativ zur Montageeinrichtung, wobei die Montagevorrichtung eine um die Montageachse einstellbare Drehkupplung zum Lagern des Verbindungsbauteils an der Montageeinrichtung bildet. Hierdurch kann zusätzlich zur einstellbaren Höhenposition auch ein Einstellen oder Nachjustieren der relativen Drehposition der Spindel relativ zur Montagevorrichtung auf einfache Weise erfolgen. Der Aktionsradius des Stativs kann auf einfache Weise festgelegt werden. Bevorzugt kann die Drehkupplung dabei ohne Demontage irgendwelcher Komponenten der Montagevorrichtung oder Stativvorrichtung eingestellt werden, abgesehen von einer Verkleidung.

Bevorzugt ist die Montageeinrichtung eingerichtet, in einem ersten Zustand das Verbindungsbauteil verdrehbar und axialfest zu lagern, und in einem zweiten Zustand das Verbindungsbauteil verdrehfest und axialfest zu lagern, insbesondere in einem mit der Justageeinrichtung gekuppelten Zustand. Dies ermöglicht in Verbindung mit der Justageeinrichtung die Einstellbarkeit von definierten Verdrehwinkelpositionen.

Als Justageeinrichtung ist dabei bevorzugt eine Einrichtung zu verstehen, mittels welcher eine bestimmte Relativposition des Verbindungsbauteils relativ zur Montageeinrichtung justiert bzw. eingestellt werden kann. Mittels der Justageeinrichtung können einzelne Relativpositionen vordefiniert werden. Die Justageeinrichtung ist bevorzugt verdrehfest am Verbindungsbauteil gelagert.

Als Drehkupplung ist dabei bevorzugt eine Verbindung zu verstehen, mittels welcher eine Kupplung in einer bestimmten Drehposition sichergestellt werden kann, wobei eine relative Drehbewegung ermöglicht werden kann, sei es schrittweise, sei es stufenlos.

Gemäß einer Variante ist die einstellbare Drehkupplung durch die Justageeinrichtung und die Montageeinrichtung gebildet, wobei die Justageeinrichtung in einer vorbestimmten Verdrehposition um die Montageachse relativ zur Montageeinrichtung positionierbar und verdrehfest lagerbar ist. Hierdurch kann ein Einstellen durch ein Verdrehen der Justageeinrichtung relativ zur Montageeinrichtung erfolgen. Die Justageeinrichtung ist eingerichtet, das Verbindungsbauteil verdrehfest an der Montageeinrichtung zu lagern.

Gemäß einer Variante ist die Justageeinrichtung separat vom Verbindungsbauteil und separat von der Montageeinrichtung als separate Komponente der Montagevorrichtung ausgebildet. Durch die separate Ausgestaltung der Justageeinrichtung von der Spindel kann eine Kupplung bereitgestellt werden, mittels welcher die Position der Spindel relativ zur Montageeinrichtung auf flexible und einfache Weise eingestellt werden kann. Bevorzugt ist die Justageeinrichtung formschlüssig mit dem Verbindungsbauteil kuppelbar, insbesondere in unterschiedlichen Axialpositionen. Diese Art Schnittstelle ermöglicht z.B. die Anordnung einer unterschiedlichen Anzahl von Trägern oder unterschiedlich dimensionierte Träger, ohne dass die Schnittstelle konstruktiv abgeändert werden muss.

Die Kavität ist geometrisch korrespondierend zum Verbindungsbauteil ausgebildet und bildet ein Drehlager für das Verbindungsbauteil. Hierdurch kann das Verbindungsbauteil in der Montageeinrichtung zwecks Justage der Drehposition verdreht werden.

Bevorzugt sind die Montageeinrichtung und die Justiereinrichtung in axialer Richtung in Reihe zueinander angeordnet und überlappen jeweils das Verbindungsbauteil.

Gemäß einer Variante weist die Justageeinrichtung einen Verdrehanschlag auf, insbesondere eine Nut oder Feder, welcher geometrisch korrespondierend zu einem am Verbindungsbauteil angeordneten Verdrehanschlag, insbesondere Nut oder Feder, ausgebildet ist. Hierdurch kann eine Drehung der Justageeinrichtung eine Drehung der Spindel bewirken und umgekehrt. Das Einstellen der Drehposition der Justageeinrichtung relativ zur Montageeinrichtung ermöglicht somit unmittelbar auch ein Einstellen der Drehposition der Spindel beispielsweise im Operationssaal. Bevorzugt erstreckt sich die Nut zumindest annähernd in Richtung einer Längsachse des Verbindungsbauteils. Der Verdrehanschlag kann wahlweise auch durch eine andere formschlüssige Kupplung sichergestellt werden, z.B. einen Zahnkranz oder irgendwelche Absätze oder Stege, die in radialer Richtung ineinander greifen. Der Verdrehanschlag kann dabei auch die Funktion einer Zentrierung übernehmen.

Bevorzugt ist die Montageeinrichtung sowohl für ein Sichern gegen Verdrehen, insbesondere mittels einer in axialer Richtung angeordneten Durchführung, als auch für ein Sichern des Verbindungsbauteils in axialer Richtung, insbesondere mittels mindestens einer in radialer Richtung angeordneten Durchführung, eingerichtet. Hierdurch kann das Nachjustieren oder Einstellen der Drehkupplung ohne weitere Hilfsmittel oder Abstützvorrichtungen auch problemlos dann erfolgen, wenn die Stativvorrichtung eine bedeutende Eigenmasse aufweist, z.B. wenn eine komplette Versorgungskonsole an der Stativvorrichtung befestigt ist.

Gemäß einer Variante weist die Justageeinrichtung eine Mehrzahl von Kupplungspunkten, insbesondere Löchern oder Durchführungen, jeweils zum Festlegen einer von einer Mehrzahl von Verdrehpositionen der Drehkupplung auf, welche bevorzugt auf einem Teilkreis angeordnet sind, wobei die Justageeinrichtung bevorzugt eine ringförmige Geometrie aufweist oder als ringförmige Scheibe (Flachring) ausgebildet ist. Die Kupplungspunkte sind jeweils bevorzugt von einer Oberseite der Justageeinrichtung zugänglich. Der Teilkreis ist bevorzugt größer als ein Durchmesser der Kavität, was eine gute Zugänglichkeit von außen sicherstellen kann. Bevorzugt sind die Kupplungspunkte möglichst weit radial außen an der Justageeinrichtung angeordnet. Hierdurch kann das Einstellen auf einfache Weise erfolgen, selbst bei schwer zugänglicher Anordnung der Justageeinrichtung unter einer Raumdecke. Bevorzugt sind die Kupplungspunkte von einer Stirnseite der Justageeinrichtung zugänglich. Die Löchern oder Durchführungen sind bevorzugt in axialer Richtung ausgerichtet, insbesondere parallel zur Montageachse.

Die Justageeinrichtung weist bevorzugt einen Außendurchmesser auf, welcher größer gleich als die weiteren Komponenten der Montagevorrichtung ist. Hierdurch kann die Justageeinrichtung eine Schnittstelle an einer Umfangsfläche oder einem Außenrand bereitstellen, insbesondere Montageschlitze, an welcher eine Verkleidung oder Abdeckung der Montagevorrichtung montiert werden kann. Die Befestigung einer Verkleidung an der Justageeinrichtung hat den Vorteil, dass die Verkleidung auf einfache Weise entfernt werden kann und dass die Drehkupplung auf einfache Weise zugänglich ist.

Die Justageeinrichtung kann eine ringförmige Auflagefläche aufweisen, welche geometrisch korrespondierend zu einer ringförmigen Lagerfläche des Verbindungsbauteils ausgebildet ist. Hierdurch kann das Verbindungsbauteil auf exakte Weise mit der Justageeinrichtung gekuppelt werden. Die Auflagefläche kann dabei als Anschlag für einen korrespondierenden Absatz des Verbindungsbauteils dienen.

Gemäß einer Variante weist die Justageeinrichtung eine Durchführung mit einem Innendurchmesser kleiner dem Durchmesser der Kavität bzw. kleiner einem Innendurchmesser einer Innenmantelfläche der Montageeinrichtung auf. Hierdurch kann das Verbindungsbauteil an einem Absatz des Verbindungsbauteils an der Justageeinrichtung zur Anlage gebracht werden.

Gemäß einer Variante weist die Montageeinrichtung eine ebene untere Stirnfläche auf, an welcher die justageeinrichtung in einer vordefinierbaren Axialposition zur Anlage bringbar ist. Hierdurch kann die Justageeinrichtung auf exakte Weise relativ zur Montageeinrichtung ausgerichtet werden, so dass ein Verdrehsicherungselement in unterschiedlichen Positionen ohne Verkanten anordenbar ist, insbesondere auf manuelle Weise.

Gemäß einer Variante weist die Montageeinrichtung mindestens einen Befestigungsabschnitt mit einer Mehrzahl von Befestigungsmitteln, insbesondere Löchern oder Bohrungen auf, wobei die Befestigungsmittel unterschiedliche axiale Montagepositionen definieren. Hierdurch kann auf einfache Weise eine spezifische Höhenposition der Stativvorrichtung relativ zu einer Raumdecke bzw. einem Deckenflansch eingestellt werden.

Bevorzugt weist die Justageeinrichtung 15 bis 30, bevorzugt 20 bis 25 Löcher auf, so dass eine relative Drehposition der Spindel relativ zur Montageeinrichtung in vergleichsweise kleinen Winkelschritten eingestellt werden kann, z.B. in Schritten von 15°. Ein solches Einstellen der relativen Drehposition ist insbesondere in Hinblick auf die Drehbewegung begrenzende Anschläge oder Verdrehsicherungen der Stativvorrichtung vorteilhaft. Somit kann der Aktionsradius der Stativvorrichtung zum Positionieren der medizintechnischen Einrichtung auf flexible Weise eingestellt werden.

Gemäß einer Variante weist die Montageeinrichtung eine Mehrzahl von Gewindebohrungen zur Aufnahme von in Längsrichtung/Höhenrichtung einbringbaren Befestigungselementen auf, wobei die Gewindebohrungen auf demselben Teilkreis angeordnet sind wie korrespondierende Kupplungspunkte/Löcher der Justageeinrichtung. Die Gewindebohrungen sind bevorzugt an einer unteren Stirnseite der Montageeinrichtung angeordnet und erstrecken sich zumindest annähernd in Längsrichtung. Dies ermöglicht eine einfache Montage und ein einfaches Nachjustieren. Bevorzugt sind drei bis fünf Gewindebohrungen vorgesehen, die im montierten Zustand der Montageinrichtung von einer Unterseite zugänglich sind, so dass Befestigungselemente in axialer Längsrichtung von unten eingesteckt und befestigt, insbesondere angeschraubt werden können. Ein Monteur kann dabei bei der Montage aller Befestigungselemente unter der Montageeinrichtung zumindest annähernd in derselben Position stehen bleiben. Eine Verschraubung in radialer Richtung ist nicht erforderlich. Dies ermöglicht auch eine Position des Monteurs weiter unten, und damit eine sicherere Montage mit weniger Risiken, z.B. weniger Risiko in Hinblick auf einen Sturz des Monteurs. Auch kann auf einfachere Weise eine Sichtprüfung der Befestigungselemente in Hinblick auf eine korrekte Position erfolgen. Ein möglicherweise in Montagerichtung, d.h. in Längsrichtung manuell aufzubringender Druck oder eine manuell aufzubringende Kraft kann leichter genau in der Längsrichtung aufgebracht werden.

Gemäß einer Variante sind die mindestens zwei Befestigungsabschnitte an einer Außenmantelfläche jeweils durch einen in radialer Richtung hervorstehenden Steg gebildet, wobei die Montageeinrichtung bevorzugt mindestens drei Befestigungsabschnitte aufweist, welche sternförmig angeordnet sind, insbesondere in gleichem Abstand in Umfangsrichtung zueinander. Hierdurch kann eine Last (insbesondere Gewichtskraft oder ein Drehmoment) an einem weit außen liegenden Kraftangriffspunkt übertragen werden. Speziell bei mehreren symmetrisch um den Umfang der Montageeinrichtung verteilten Befestigungsabschnitten kann eine Kraft homogen verteilt an vorteilhaften Kraftangriffspunkten übertragen werden. Auch kann für den Fall, dass die Justageeinrichtung an der Montageeinrichtung befestigt werden soll, eine bestimmte Drehposition auf vergleichsweise genaue Weise oder in besonders kleinen Dreh-Schritten oder Dreh-Winkeln eingestellt werden, insbesondere unabhängig von einem Durchmesser der Spindel und weitgehend unabhängig von den zu übertragenden Lasten.

Bevorzugt sind die Befestigungsabschnitte umlaufend an der Außenmantelfläche angeordnet, insbesondere in demselben Abstand in Umfangsrichtung zueinander. Dies erleichtert ein Einstellen in Hinblick auf eine Vielzahl unterschiedlicher Drehwinkel-Positionen.

Gemäß einer Variante weist die Montageeinrichtung eine insbesondere rohrartige Aufnahme für ein Verdrehsicherungselement auf, wobei die Aufnahme bevorzugt an einer Außenseite, insbesondere Außenmantelfläche der Montageeinrichtung angeordnet ist oder die Außenmantelfläche zumindest abschnittsweise bildet. Hierdurch kann das Verdrehsicherungselement auf einfache Weise entfernt oder eingesetzt werden. Die Aufnahme weist bevorzugt eine Durchgangsbohrung auf, welche bis zu einer unteren Stirnseite der Montageeinrichtung führt. Eine derart ausgestaltete Aufnahme kann auch als Sicherungsrohr bezeichnet werden.

Bevorzugt ist die Aufnahme zur Anordnung eines in axialer Richtung ausgerichteten Verdrehsicherungselements ausgebildet, insbesondere einer Bolzenverbindung in axialer Richtung. Die Aufnahme umfasst dabei bevorzugt einen unteren (insbesondere rohrartigen) Aufnahmeabschnitt, welcher eine axiale Erstreckung kleiner einer Länge des Verdrehsicherungselements aufweist.

Gemäß einer Variante weist die Aufnahme einen Zugang auf, insbesondere einen Zugang von radial außen, wobei der Zugang bevorzugt in Form einer Ausfräsung ausgebildet ist. Hierdurch wird eine manuelle Justage erleichtert. Bevorzugt weist der Zugang in axialer Richtung eine Erstreckung auf, welche größer ist als eine Länge eines Verdrehsicherungselements und größer als eine axiale Erstreckung eines unteren Abschnitts der Aufnahme.

Der Zugang weist bevorzugt eine oberseitige Zugangsfläche auf, an welcher ein Verdrehsicherungselement zur Anlage gebracht werden kann. An der oberseitigen Zugangsfläche kann z.B. ein Bolzen mit einem Rand/Kopf/Absatz gelagert werden, so dass die Drehkupplung auf einfache Weise manuell eingestellt werden kann. Der Bolzen kann in die Aufnahme gesteckt werden und allein durch Gravitationskräfte darin gesichert werden. Hierdurch muss ein Monteur nur eine Steckbewegung ausführen, welche auch in besonders kurzer Zeit ausgeführt werden kann. Eine Drehbewegung oder ein Verschrauben ist nicht erforderlich, was insbesondere an schlecht zugänglichen Positionen direkt unterhalb einer Raumdecke vorteilhaft ist. Das Verdrehsicherungselement kann manuell auf einfache Weise demontiert und wieder montiert werden, insbesondere werkzeuglos. Dies liefert gerade an schwer zugänglichen Aufhängungspunkten der Stativvorrichtung Vorteile, nicht zuletzt weil ein Monteur beide Hände frei hat.

Gemäß einer Variante weist die Montagevorrichtung eine Axialsicherung auf, mittels welcher das Verbindungsbauteil in einer vordefinierten Axialposition an der Montageeinrichtung lagerbar ist, insbesondere relativ verdrehbar zur Montageeinrichtung. Die Axialsicherung kann die Montage oder auch das Nachjustieren erleichtern. Das Stativ kann mittels der Axialsicherung gesichert werden, insbesondere während der Justage der Drehposition, oder während dem Befestigen von einzelnen Befestigungselementen. Die Montagevorrichtung kann auch ein Risiko eines Verkantens innerhalb der Montageeinrichtung vermindern. Die Montagevorrichtung kann dabei mit nur drei Hauptkomponenten eine auf einfache Weise einstellbare Drehkupplung bereitstellen. Die Kupplung besteht dabei aus drei Hauptkomponenten, nämlich der Montageeinrichtung, der Justageeinrichtung bzw. dem Flachring sowie der Axialsicherung.

Bevorzugt bildet die Axialsicherung in Verbindung mit der Kavität ein axialfestes Drehlager für das Verbindungsbauteil, also ein Lager, bei welchem eine Verdrehung in einer vordefinierten Axialposition möglich ist. Das axialfeste Drehlager liefert dabei einen Bewegungsfreiheitsgrad um die Montageachse und verhindert eine Bewegung entlang der Montageachse.

Gemäß einer Variante ist die Axialsicherung eingerichtet, dauerhaft montiert zu sein und die axiale Sicherung des Verbindungsbauteils in unterschiedlichen bzw. allen Montagesituationen sicherzustellen.

Gemäß einer Variante ist die Montageeinrichtung zum Halten und verdrehbaren Lagern des Verbindungsbauteils in einer vorbestimmten axialen Relativposition ausgebildet, wobei an einer die Kavität begrenzenden Innenmantelfläche der Montageeinrichtung eine Fase, Kante oder Einfräsung vorgesehen ist. Hierdurch kann durch einfach aufgebaute konstruktive Komponenten ein axialfestes Drehlager gebildet werden, welches bei einer Montage der Stativvorrichtung auf einfache Weise montierbar ist.

Gemäß einer Variante umfasst die Axialsicherung eine tangential an der Montageeinrichtung angeordnete Durchführung, welche eine Außenkontur oder Außenmantelfläche der Montageeinrichtung durchstößt, bevorzugt an zwei Punkten, und welche eine/die Innenmantelfläche der Montageeinrichtung schneidet. Die Durchführung (insbesondere Bohrung oder Ausfräsung) kann wie eine Sekante relativ zur Außenmantelfläche der Montageeinrichtung angeordnet sein. In die Durchführung kann ein tangential zum Verbindungsbauteil angeordneter Sicherungselement (insbesondere Bolzen oder Riegel) eingebracht werden, welches in eine Nut des Verbindungsbauteils eingreifen kann und das Verbindungsbauteil axial in der Montageeinrichtung sichern kann. Bevorzugt ist das Sicherungselement tangential zur Montageeinrichtung anordenbar und geometrisch korrespondierend zur Durchführung ausgebildet.

Die tangential ausgerichtete Durchführung kann eine Innenkontur, insbesondere Innenmantelfläche der Kavität derart schneiden, dass der Riegel weiter innen angeordnet ist als die Innenmantelfläche. Beispielsweise ragt der Riegel um die Hälfte seines Durchmessers weiter nach innen als die Innenmantelfläche. Eine als Sekante angeordnete Durchführung liefert den Vorteil eines z.B. im Vergleich zu einem radial ausgerichteten Sicherungsbolzens vergleichsweise langen Eingriffsabschnitt des Riegels mit dem Verbindungsbauteil. Der Riegel ist dabei tangential zum Verbindungsbauteil angeordnet und greift an einer Umfangsseite des Verbindungsbauteils abschnittsweise tangential in das Verbindungsbauteil ein. Diese Art einer Axialsicherung hat auch den Vorteil, dass die Axialsicherung nicht entfernt werden muss, insbesondere beim Verdrehen des Verbindungsbauteils während der Montage. Eine Reibung bei relativer Drehung des Verbindungsbauteils innerhalb der Kavität kann dabei vergleichsweise klein gehalten werden. Ein Verdrehen kann selbst bei langen Tragarmen bzw. hohen Gewichten oder Momenten auf einfache Weise erfolgen, insbesondere durch Eingriff in eine Aussparung an der Unterseite der Justageeinrichtung. Die Axialsicherung bzw. der Tangential-Riegel kann in der tangentialen Position positioniert bleiben.

Gemäß einer Variante können zwischen der Justageeinrichtung und der unteren Stirnseite der Montageeinrichtung Distanzscheiben vorgesehen sein, um eine Höhenposition des Verbindungsbauteils relativ zur Montageeinrichtung einstellen zu können. Auch können mehrere tangentiale Durchführungen jeweils zur Aufnahme eines Sicherungselements zur Axialsicherung übereinander in der Montageeinrichtung vorgesehen sein. Hierdurch kann auf einfache Weise eine Höhenverstellung erfolgen, wobei Distanzscheiben nicht notwendigerweise erforderlich sind.

Bevorzugt ist an der Durchführung eine Auflagefläche ausgebildet, welche eingerichtet ist, eine vom Verbindungsbauteil ausgeübte Gewichtskraft auf die Montageeinrichtung zu übertragen. Gemäß einer Variante ist die Auflagefläche U-förmig. Hierdurch lassen sich die Durchführungen auf wirtschaftliche Weise herstellen, insbesondere durch Fräsen. Andererseits können sowohl in der Montageeinrichtung als auch im Verbindungsbauteil Kerbspannungen minimal gehalten werden. Bevorzugt weist die Durchführung auf der oberen Seite kleinere Radien auf als auf der Unterseite. Dadurch kann vorgegeben werden, in welcher Anordnung das jeweilige Sicherungselement in der Durchführung angeordnet werden soll. Ein Risiko einer fehlerhaften Montage kann dadurch verringert werden.

Gemäß einer Variante umfasst die Axialsicherung mindestens ein Sicherungselement, insbesondere einen Riegel, welches ausgelegt und eingerichtet ist, die Gewichtskraft der Stativvorrichtung vom Verbindungsbauteil auf die Montageeinrichtung zu übertragen. Hierdurch kann ein Nachjustieren der Drehposition erfolgen, ohne irgendwelche Komponenten der Stativvorrichtung, insbesondere irgendwelche Komponenten einer Zentralachse, demontieren zu müssen. Die gesamte Stativvorrichtung kann während des Einstellens der Drehposition an der Axialsicherung gelagert werden. Mit anderen Worten ist die Axialsicherung eingerichtet, ein Drehlager für die Stativvorrichtung zu bilden. Dies erleichtert das Nachjustieren erheblich, denn lediglich die Justageeinrichtung braucht gelöst und wieder in einer nachjustierten Drehposition befestigt zu werden.

Gemäß einer Variante sind die Durchführung und die Aufnahme für ein Verdrehsicherungselement an demselben Umfangsflächenabschnitt der Montageeinrichtung angeordnet, insbesondere von derselben Seite manuell zugänglich. Dies erleichtert das Einstellen und Montieren der Vorrichtung. Dies erleichtert das Einstellen und Montieren der Vorrichtung. Dabei kann eine Sicherung des Verdrehsicherungselements und der Sicherungselemente zur Axialsicherung mittels Federsteckern erfolgen, und zwar von derselben Seite bzw. am selben Umfangsflächenabschnitt. Dies liefert nicht zuletzt Vorteile bei der Montage.

Das Montagesystem kann dabei auch das Verbindungsbauteil in Form einer Spindel sowie ein Verdrehsicherungselement und mindestens ein Sicherungselement zur Axialsicherung aufweisen, wobei an der Spindel eine umlaufende Nut oder ein umlaufender Absatz vorgesehen ist, welche/r geometrisch korrespondierend zum Sicherungselement ausgebildet ist. Mit anderen Worten kann das Montagesystem unterschiedliche Sicherungselemente jeweils zur Axialsicherung und zur Verdrehsicherung umfassen. Bevorzugt sind beide Typen der Sicherungselemente an derselben Umfangsposition der Montageeinrichtung montierbar. Dabei kann ein einziges Sicherungselement zur Axialsicherung vorgesehen sein. Bevorzugt sind zwei Sicherungselemente zur Axialsicherung vorgesehen. Wahlweise können auch drei Sicherungselemente zur Axialsicherung vorgesehen sein.

Bevorzugt ist die Nut umlaufend vorgesehen und erstreckt sich orthogonal zur Längsrichtung. Eine umlaufende Nut liefert den Vorteil, dass der Bolzen der Axialsicherung unabhängig von einer spezifischen Drehposition in Eingriff mit der Spindel gebracht werden kann. Auch kann die Spindel auf einfache Weise relativ zur Montageeinrichtung gedreht werden, selbst bei einer auf die Axialsicherung in axialer Richtung einwirkenden Last. Dies erleichtert das Nachjustieren oder Umstellen der Drehposition.

Bevorzugt ist die Nut in einem Abstand zu einer Stirnseite oder einem stirnseitigen Anschlag der Spindel angeordnet, welcher in Längsrichtung gesehen einem Abstand der Bohrung von einem Gegenanschlag in der Kavität entspricht. Dies erleichtert die Montage, insbesondere da die Spindel mit der Stirnseite auf einfache Weise an einem/dem Gegenanschlag der Montageeinrichtung derart zur Anlage gebracht werden kann, dass die Spindel in der richtigen Axialposition angeordnet ist, um die Axialsicherung vorzusehen. In dieser Axialposition kann der Bolzen tangential an einer Außenmantelfläche der Spindel eingreifen. Ein Nachjustieren der Axialposition ist nicht erforderlich. Der Gegenanschlag der Montageeinrichtung kann dabei auch durch einen Boden der Kavität oder einen umlaufenden ringförmigen oder scheibenförmigen Gegenanschlag am Boden der Kavität bereitgestellt werden.

Gemäß einer Variante ist an der Spindel ein Absatz ausgebildet, welchen die Justageeinrichtung in radialer Richtung überlappt. Dies ermöglicht eine Lagerung der Spindel mittels der Justageeinrichtung. Dieser Absatz kann ermöglichen, die Justageeinrichtung zusammen einem oder mehreren Tragarmen über eine die Tragarme sichernde untere Wellenmutter zu fixieren. Die Montage kann insbesondere dadurch erleichtert werden, dass die Justageeinrichtung vor dem Einschieben der Spindel in die Montageeinrichtung gesichert wird, speziell in Hinblick auf ein Verrutschen nach unten.

Zur Montage kann zunächst die Montageeinrichtung an der Raumdecke montiert werden. Dann kann die Spindel von unten in die Kavität der Montageeinrichtung gesteckt werden. Die Justageeinrichtung ist dabei bevorzugt bereits auf der Spindel angeordnet und kann an der Montageeinrichtung befestigt werden. Hierdurch kann auch die Spindel in axialer Längsrichtung an der Montageeinrichtung positioniert werden. Wahlweise kann die Spindel vor dem Befestigen der Justageeinrichtung auch mittels einer Axialsicherung an der Montageeinrichtung gesichert werden, so dass die Justageeinrichtung in einer bestimmten Drehposition positioniert werden kann, ohne dass gleichzeitig eine Gewichtskraft der Spindel mittels der Justageeinrichtung aufgenommen werden muss. Dies erleichtert die Montage oder auch ein nachträgliches Einstellen einer bestimmten Drehposition.

Gemäß einer Variante hat der Absatz der Spindel einen Außendurchmesser, der größer ist als der Innendurchmesser einer Durchführung der Justageeinrichtung. Bei dieser Ausgestaltung ist die Spindel wahlweise auch mittels der Justageeinrichtung in axialer Richtung lagerbar. Die Überlappung in radialer Richtung beträgt bevorzugt mindestens 1mm.

Gemäß einer Variante ist unterhalb des Absatzes ein Verdrehanschlag, insbesondere eine (Paß-)Feder, angeordnet, der/die bevorzugt in Längsrichtung ausgerichtet ist. Der Verdrehanschlag ist geometrisch korrespondierend zu einem Verdrehanschlag der Justageeinrichtung ausgebildet. Der Verdrehanschlag kann z.B. eine in einer entsprechenden Nut auf der Spindel montierte Paßfeder sein, oder eine angegossene Feder.

Gemäß einer Variante umfasst das Montagesystem ein Verdrehsicherungselement in Form eines in axialer Richtung eingreifenden Bolzens sowie zwei Sicherungselemente in Form von in tangentialer Richtung eingreifenden Riegeln, wobei der Bolzen und die Riegel angrenzend an einen in einer Aufnahme für das Verdrehsicherungselement vorgesehenen Zugang angeordnet sind. Diese Anordnung erleichtert das Einstellen und die Montage.

Auch offenbart (nicht beansprucht) wird ein Halter für eine im Operationssaal anordenbare Stativvorrichtung, wobei der Halter eingerichtet ist, ein Verbindungsbauteil der Stativvorrichtung mit einem Deckenflansch zu verbinden, wobei der Halter eingerichtet ist, das Verbindungsbauteil in unterschiedlichen Höhenpositionen entlang einer Montageachse an den Deckenflansch zu kuppeln, wobei der Halter eine Kavität oder wenigstens eine Backe aufweist, welche eine Kupplung oder Führungsfläche zum Einstellen der Höhenpositionen bildet. Damit ergeben sich bereits zuvor beschriebene Vorteile.

Auch offenbart (nicht beansprucht) wird eine Montageeinrichtung für eine im Operationssaal anordenbare Stativvorrichtung, wobei sich die Montageeinrichtung in Höhenrichtung entlang einer Montageachse erstreckt und zum Halten eines Verbindungsbauteils der Stativvorrichtung eingerichtet ist, wobei die Montageeinrichtung mindestens einen Befestigungsabschnitt mit einer Mehrzahl von insbesondere identisch ausgebildeten Befestigungsmitteln aufweist, welche in Höhenrichtung beabstandet zueinander angeordnet sind, wobei der mindestens eine Befestigungsabschnitt durch einen an einer Außenseite, Außenkontur oder Außenmantelfläche der Montageeinrichtung angeordneten Steg gebildet ist, insbesondere durch einen in radialer Richtung orthogonal zur Höhenrichtung abstehenden Steg. Damit ergeben sich bereits zuvor beschriebene Vorteile.

Auch offenbart (nicht beansprucht) wird die Verwendung eines Halters und/oder einer Montageeinrichtung, wie zuvor beschrieben, jeweils an einer im Operationssaal angeordneten Stativvorrichtung, wobei der Halter und/oder die Montageeinrichtung unterschiedliche Höhenpositionen entlang einer Montageachse festlegt und die Stativvorrichtung in einer spezifischen der Höhenpositionen anordnet. Hierdurch ergeben sich bereits zuvor beschriebene Vorteile.

In den nachfolgenden Zeichnungsfiguren wird die Erfindung anhand von Ausführungsbeispielen näher erläutert. Es zeigen:
- Figur 1: in perspektivischer Seitenansicht eine Montagevorrichtung gemäß einem Ausführungsbeispiel der Erfindung in einer montierten Anordnung an einer Stativvorrichtung;
- Figur 2: in perspektivischer geschnittener Seitenansicht die in Fig. 1 gezeigte Montagevorrichtung;
- Figur 3: in perspektivischer Seitenansicht eine Justageeinrichtung der in der Fig. 1 gezeigten Montagevorrichtung in Draufsicht, wobei die Justageeinrichtung um eine Spindel der Stativvorrichtung angeordnet ist;
- Figur 4: in Draufsicht von einer Unterseite aus betrachtet einzelne Komponenten der in Fig. 1 gezeigten Montagevorrichtung;
- Figur 5: in perspektivischer Seitenansicht einzelne Komponenten der in Fig. 1 gezeigten Montagevorrichtung;
- Figur 6: in perspektivischer Seitenansicht eine Montageeinrichtung der in Fig. 1 gezeigten Montagevorrichtung;
- Figur 7: in perspektivischer Ansicht von einer Unterseite die in der Fig. 1 gezeigte Justageeinrichtung;
- Figur 8: in perspektivischer Ansicht von oben die in der Fig. 7 gezeigte Justageeinrichtung;
- Figur 9: in perspektivischer Ansicht die in der Fig. 3 gezeigte Spindel;
- Figur 10: in perspektivischer Ansicht ein Sicherungselement der in Fig. 1 gezeigten Montagevorrichtung;
- Figuren 11A, 11B: in perspektivischer Ansicht sowie in Draufsicht einen Halter der in Fig. 1 gezeigten Montagevorrichtung;
- Figur 12: in perspektivischer Ansicht eine Montagevorrichtung gemäß einem weiteren Ausführungsbeispiel der Erfindung in einer montierten Anordnung an einer Stativvorrichtung mit montierten Sicherungselementen;
- Figur 13: in perspektivischer Ansicht ein Verdrehsicherungselement, welches zur Sicherung einer Drehposition der Montagevorrichtung eingerichtet ist;
- Figur 14: in geschnittener Seitenansicht ein weiteres Ausführungsbeispiel einer Montagevorrichtung; und
- Figur 15: eine Spindel speziell für die in Figur 14 gezeigte Montagevorrichtung.

Im Zusammenhang mit der Beschreibung der folgenden Figuren wird bei Bezugszeichen, falls sie bei einzelnen Figuren nicht explizit erläutert werden, auf die weiteren Figuren verwiesen.

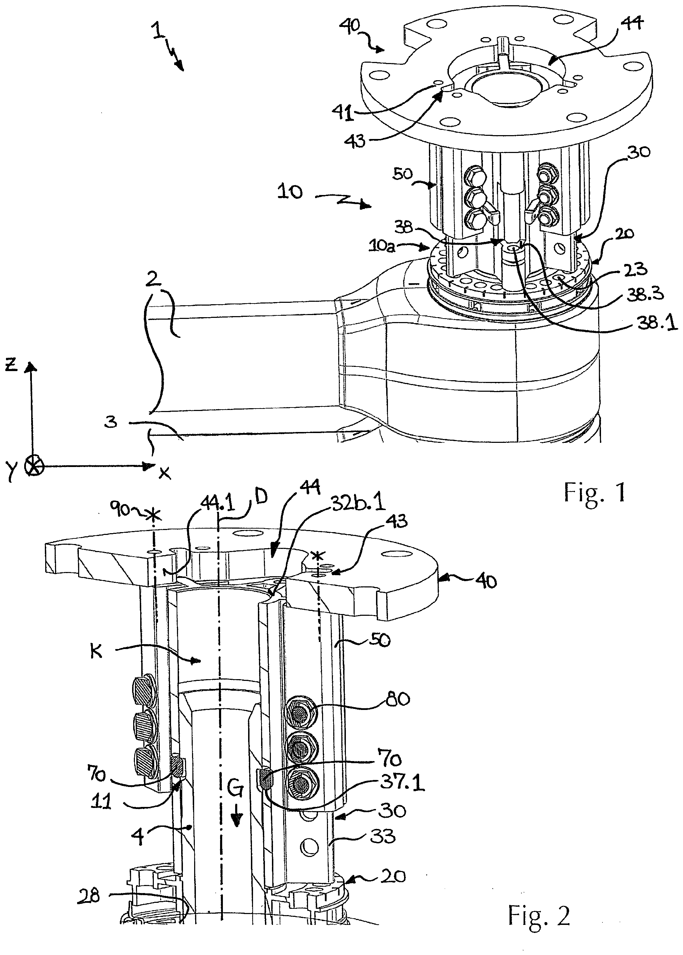

In der Figur 1 ist eine Stativvorrichtung für eine Deckenmontage gezeigt. Die Stativvorrichtung 1 umfasst einen ersten Träger 2 und einen zweiten Träger 3, welche drehbar um eine Montageachse übereinander in einem Drehgelenk gelagert sind. Für die Montage der Stativvorrichtung 1 an einer (Zwischen-)Decke sind die beiden Träger 2, 3 mit einer Montagevorrichtung 10 verbunden bzw. indirekt verkuppelt, welche einen anpassbaren Mechanismus 10a in Form einer einstellbaren Drehkupplung aufweist. Die Stativvorrichtung 1 kann mittels eines Deckenflansch 40, insbesondere in der Art einer Flanschplatte, an einer Decke oder Zwischendecke montiert werden. Der Deckenflansch 40 weist Befestigungsmittel 41 in Form von Öffnungen, insbesondere (Innengewinde-)Bohrungen auf, durch welche hindurch Befestigungsmittel, wie z.B. Schrauben montierbar sind. Die Montage erfolgt mittels Haltern 50, welche eine Verbindung des Deckenflansch 40 zu einer Montageeinrichtung 30 sicherstellen. Die Montageeinrichtung 30, ist insbesondere rohrförmig ausgebildet und weist radial von ihrer Aussenkontur 31 abstehende, als Stege ausgebildete Befestigungsabschnitte 33 auf. Es sind wenigstens zwei, vorliegend genau drei Befestigungsabschitte 33 an der Montageeinrichtung 30 vorgesehen, die mit einer korrespondierenden Anzahl an Haltern 50, zusammenwirken, die an dem Deckenflansch 40 festgelegt sind und die nachfolgend noch genauer beschrieben werden. Die Montageeinrichtung 30 wirkt mit einer Justageeinrichtung 20 zusammen. Die Justageeinrichtung 20 kann als Flachring beschrieben werden. Hierzu weist die Montageeinrichtung 30 eine Verdrehsicherung auf, insbesondere eine Aufnahme 38 in Form eines rohrartigen Abschnitts zur Aufnahme eines Sicherungselements. An einer oberseitigen Zugangsfläche 38.3 kann durch eine Durchgangsbohrung 38.1 ein Bolzen oder Stift gesteckt werden, welcher mit einem spezifischen Kupplungspunkt 23 der Justageeinrichtung zusammenwirkt. Mehrere Kupplungspunkte 23 definieren dabei jeweils eine spezifische Verdrehposition. Die Kupplungspunkte 23 können z.B. als Löchern oder Bohrungen oder seitlich offene Schlitze oder Aussparungen ausgebildet sein.

Dabei deutet ein Koordinatensystem die horizontale und bei einer Deckenmontage gleichzeitig auch radiale x-Richtung sowie die vertikale z-Richtung an.

Die Justiereinrichtung 20 kann dabei z.B. auf einem Innenring eines im Träger 2 angeordneten Kugellagers auf dem Träger 2 gelagert sein.

In der Figur 2 ist gezeigt, auf welche Weise der Deckenflansch 40 mit der Montageeinrichtung 30 verbunden werden kann. Der Deckenflansch 40 weist mehrere Radialnuten 43 auf, welche geometrisch korrespondierend zu den als Stegen ausgebildeten Befestigungsabschnitten 33 der Montageeinrichtung 30 ausgebildet sind. Ferner weist der Deckenflansch 40 eine Durchführung 44 auf, welche geometrisch korrespondierend zur Montageeinrichtung 30 ausgebildet ist, so dass die Montageeinrichtung 30 im Deckenflansch 40 gelagert oder relativ zum Deckenflansch 40 verlagert werden kann. Die Durchführung 44 ist durch eine Innenmantelfläche 44.1 gebildet, welche zumindest annähernd dieselbe Umfangslinie oder Querschnittsgeometrie aufweist wie eine Außenmantelfläche 31 der Montageeinrichtung. Bevorzugt ist die Querschnittsgeometrie der Innenmantelfläche 44.1 etwas weiter (im Durchmesser größer) als die Querschnittsgeometrie der Außenmantelfläche 31. Die Innenmantelfläche 44.1 kann ein Gleitlager oder eine Gleitfläche für die Montageeinrichtung bilden und dazu dienen, die Montageeinrichtung 30 relativ zum Deckenflansch 40 auszurichten oder zu zentrieren. Die Innenmantelfläche 44.1 kann dabei auch die Montage erleichtern, insbesondere weil Befestigungsmittel auf einfache Weise zueinander fluchtend angeordnet werden können.

Dabei kann der Deckenflansch 40 mittels Befestigungselementen 90, insbesondere Schrauben, so an den Haltern 50 befestigt werden, dass eine Gewichtskraft G von einem in der Montageeinrichtung 30 gelagerten Verbindungsbauteil 4, insbesondere einer Spindel, über die Montageeinrichtung 30, die Halter 50 und die Befestigungselemente 90 auf der Deckenflansch 40 und damit auf eine Decke oder Zwischendecke übertragen werden kann. Die Spindel 4 ist in der durch die Montageeinrichtung 30 gebildeten Kavität K gelagert. Dabei ist eine relative Verdrehung der Spindel 4 relativ zur Montageeinrichtung 30 möglich. Die gezeigte Anordnung ist um eine sich in z-Richtung erstreckende Montageachse D drehbar gelagert. Die relative Verdrehbarkeit kann mittels der Justageeinrichtung 20 verhindert werden.

Eine axiale Relativbewegung bzw. Verlagerung nach unten wird dadurch verhindert, dass eine Axialsicherung 11 vorgesehen ist, an welcher die Spindel 4 zur Anlage kommt. Im gezeigten Beispiel ist die Axialsicherung 11 durch mehrere Sicherungselemente 70, insbesondere Riegel, gebildet, welche in tangentialer Richtung in Bezug auf eine Montageachse D angeordnet sind und sowohl in die Spindel 4 als auch in die Montageeinrichtung 30 eingreifen.

Die Montageeinrichtung 30 kann in unterschiedlichen axialen Relativpositionen (Höhenpositionen) relativ zu den Haltern 50 bzw. zum Deckenflansch 40 montiert werden, nämlich mittels Befestigungselementen 80, insbesondere Schrauben, welche in entsprechende Löcher oder Durchgangsbohrungen am jeweiligen Befestigungsabschnitt 33 bzw. am jeweiligen Halter 50 eingreifen.

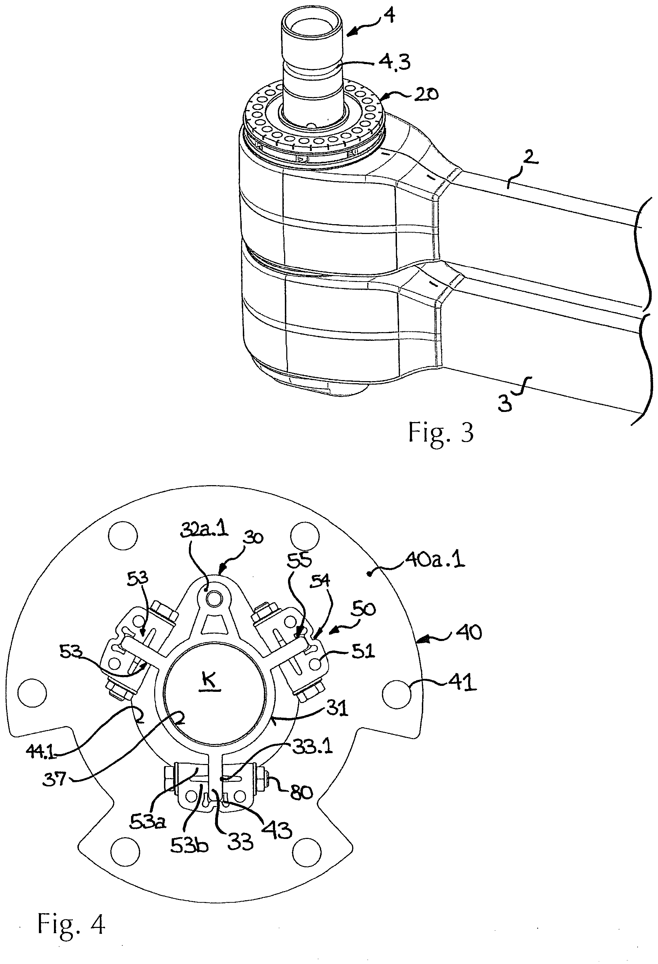

In der Figur 3 ist die Spindel 4 in einer Form gezeigt, in welcher sie von unten in die Kavität K der Montageeinrichtung 30 eingesetzt werden kann. Dabei können die zuvor beschriebenen Sicherungselemente 70 in der Endposition mit einer umlaufenden Nut 4.3 zusammenwirken. Die Spindel 4 ist dann an der Nut 4.3 verdrehbar und axialfest in der Kavität K gelagert.

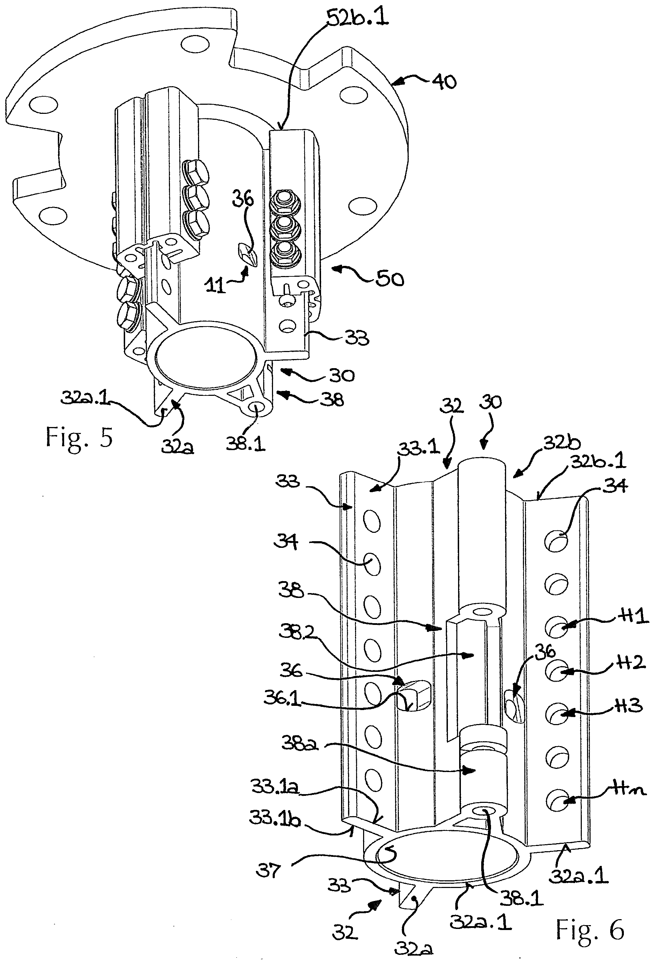

In der Figur 4 ist eine ebene unterseitige Stirnfläche 42a.1 des Deckenflansch 40 gezeigt, an welcher der jeweilige Halter 50 mit einer ebenen oberseitigen Stirnfläche 52.b.1 (angedeutet in Figur 5) zur Anlage kommen kann. Der jeweilige Halter 50 weist durchgehende Befestigungsmittel 51 auf, insbesondere Innengewindebohrungen, in welche z.B. Schrauben eingreifen können. Der jeweilige Befestigungsabschnitt 33 kommt an gegenüberliegenden Backen 53 zur Anlage. Die Backen können mittels der Schrauben 80 an eine entsprechende Radialflanke 33.1 gepresst werden. Jede Backe 53 weist einzelne Backenabschnitte 53a, 53b auf, welche eine sichere oder belastbare Verbindung des jeweiligen Halters 50 mit der Montageeinrichtung 30 sicherstellen können.

Ferner weist der jeweilige Halter 50 eine Wellung 54 auf, welche eine nach außen weisenden Fläche zumindest abschnittsweise bildet, und dank welcher der Halter 50 auf vergleichsweise einfache bzw. flexible Weise aufgebogen und problemlos am jeweiligen Befestigungsabschnitt 33 montiert werden kann.

Die gegenüberliegenden Backen 53 definieren einen Befestigungsabschnitt 55 in Form einer Radialkavität, insbesondere in Schlitzform, welche geometrisch korrespondierend zum jeweiligen Befestigungsabschnitt 33 ausgebildet ist. Der Befestigungsabschnitt 55 ist in radialer Richtung radial außen durch eine Zentrierfläche 54.1 begrenzt, an welcher ein Umfangsflächenabschnitt des entsprechenden Befestigungsabschnitts 33 der Montageeinrichtung 33 zur Anlage kommen kann. Dabei erstrecken sich die Befestigungsabschnitte 33 in radialer Richtung nach außen von einer (abschnittsweise) zylindrischen Außenmantelfläche 31 und bilden diese Außenmantelfläche weiter fort. Die Montageeinrichtung 30 weist eine zylindrische Innenmantelfläche 37 auf, durch welche die Kavität K definiert bzw. in radialer Richtung begrenzt ist.

In der Figur 5 ist die Montageeinrichtung 30 in einem am Deckenflansch 40 montierten Zustand von einer Unterseite gezeigt. Sowohl die Aufnahme 38 für eine Verdrehsicherung als auch die einzelnen Befestigungsabschnitte 33 definieren eine untere Stirnseite 32a, welche durch eine ebene untere Stirnfläche 32a.1 gebildet ist. An dieser Stirnfläche 32a.1 kann die Justageeinrichtung zur Anlage kommen. Die Montageeinrichtung 30 kann dabei z.B. als Stranggussprofil ausgebildet sein. An der Mantelfläche der Montageeinrichtung 30 sind eine oder mehrere Durchführungen 36, insbesondere in tangentialer Richtung, eingebracht, welche einen Teil der Axialsicherung 11 bilden.

In der Figur 6 ist die Montageeinrichtung 30 isoliert dargestellt. Die Montageeinrichtung 30 ist als Stranggussprofil ausgebildet und weist gegenüberliegende Stirnseiten 32 auf. Ebenso wie die untere Stirnseite 32a ist eine obere Stirnseite 32b durch eine ebene obere Stirnfläche 32b.1 gebildet. Entlang eines jeden Befestigungsabschnitts 33 sind eine Vielzahl von Löchern oder Befestigungsmitteln 34 vorgesehen, z.B. Gewindebohrungen oder Bohrungen. Die Durchführungen 36 definieren jeweils eine Auflagefläche 36.1, auf welcher das in Fig. 2 gezeigte Sicherungselement 70 zur Anlage kommen kann. Die Auflagefläche 36.1 kann dabei zumindest teilweise auch durch eine an der Innenmantelfläche 37 gebildete Fase, Kante oder Einfräsung 37.1 gebildet sein (vgl. Fig. 2). Ein Kraftflusspfad einer zu übertragenden Gewichtskraft verläuft durch diese Auflageflächen 36.1. Die Durchführungen 36 grenzen radial innen an mindestens zwei der Befestigungsabschnitte 33 an. Jeder Befestigungsabschnitt 33 weist gegenüberliegende, insbesondere parallel zueinander ausgerichtete Radialflanken 33.1a, 33.1b auf. Die Verdrehsicherung 38 bzw. die Aufnahme ist in Form eines Sicherungsrohrabschnitts ausgebildet. Die Verdrehsicherung 38 weist einen unteren rohrartigen Aufnahmeabschnitt 38a auf. Der untere Aufnahmeabschnitt 38a kann unterteilt sein, insbesondere durch einen Schlitz, wodurch ein zusätzliches Sichern eines/des Verdrehsicherungselements mittels eines Federsteckers ermöglicht werden kann. Die Aufnahme 38 weist einen Zugang 38.2 in Form einer Ausfräsung auf. An diesem Zugang oder im Bereich dieses Zugangs kann ein Bolzen in die Durchgangsbohrung 38.1 am unteren Aufnahmeabschnitt 38a gesteckt werden, nämlich von oben in eine an der unteren Stirnfläche 32a.1 angeordneten Justageeinrichtung.

Die Befestigungsmittel 34 definieren unterschiedliche Höhenpositionen H1, H2, H3, ..., Hn, entsprechend Montagepunkten, an welchen jeweils Befestigungselemente befestigt oder gelagert werden können. Bei einer Befestigung untersten Montagepunkt Hn, welcher am nahesten zur Unterseite der Montageeinrichtung 30 angeordnet ist, kann die Montageeinrichtung 30 bzw. Stativvorrichtung in einer höchsten Höhenposition angeordnet werden.

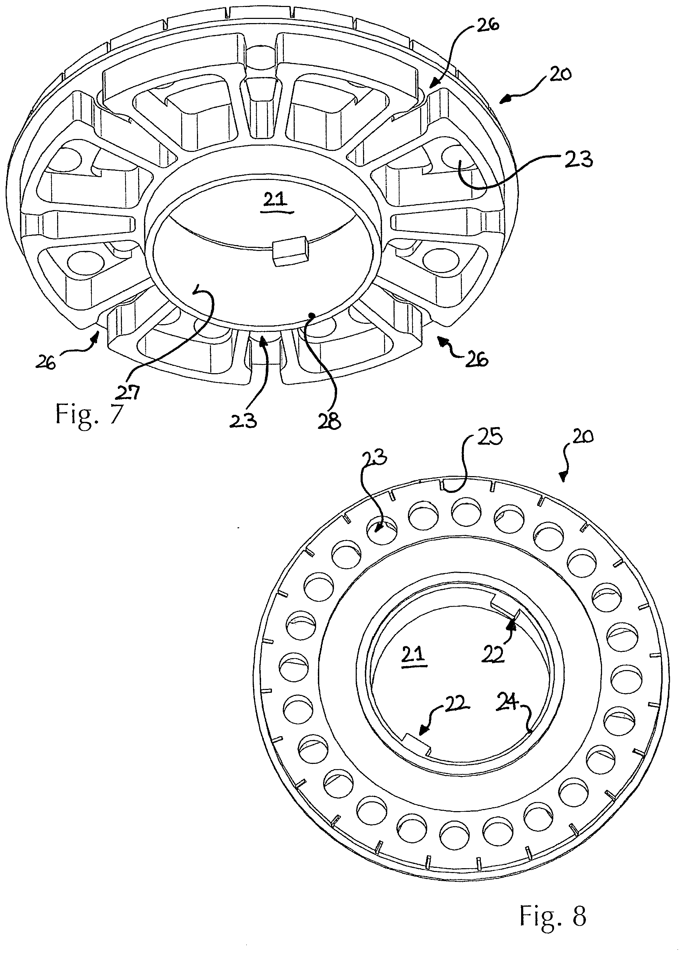

In der Figur 7 ist die Justageeinrichtung 20 von einer Unterseite gezeigt. Die Justageeinrichtung 20 kann als Flachring beschrieben werden. Die nicht dargestellte Spindel kann durch eine Durchführung 21 hindurchgeführt werden. An einer Innenmantelfläche 27 ist ein Verdrehanschlag 22 (wahlweise auch in der Funktion einer Zentrierung) angeordnet, insbesondere in Form einer Feder, welche geometrisch korrespondierend zu einer entsprechenden Nut der Spindel ausgebildet ist. Bevorzugt sind zwei Federn 22 vorgesehen, wie in Figur 8 gezeigt. Die Federn 22 sind an einem oberen Rand der Justageeinrichtung 20 angeordnet. An der Unterseite weist die Justageeinrichtung 20 einen hervorstehenden Rand 28 auf, welcher dank einer vergleichsweise großen Axialerstreckung eine exakte Ausrichtung oder Zentrierung gegenüber der Spindel erleichtert. An der Unterseite der Justageeinrichtung 20 sind Aussparungen 26 oder Radialschlitze vorgesehen, in welche ein Monteur mit einem Werkzeug (z.B. einem Schraubendreher) eingreifen kann, insbesondere von radial außen, um die Justageeinrichtung 20 und damit die Spindel zu verdrehen. Auf diese Weise kann die relative Verdrehposition justiert werden, insbesondere ohne Demontage irgendeiner Verkleidung oder irgendeines Gehäuses. Wie bereits in Bezug auf Fig. 1 beschrieben, weist die Justageeinrichtung eine Vielzahl von Kupplungspunkten 23 auf, welche geometrisch korrespondieren zu einem Mittel zur Verdrehsicherung, insbesondere einem Bolzen, ausgebildet sind. Gemäß einer Variante sind die Kupplungspunkte 23 als Löcher ausgebildet und weisen zumindest annähernd denselben Durchmesser auf wie die in Figur 6 gezeigte Durchgangsbohrung 38.1.

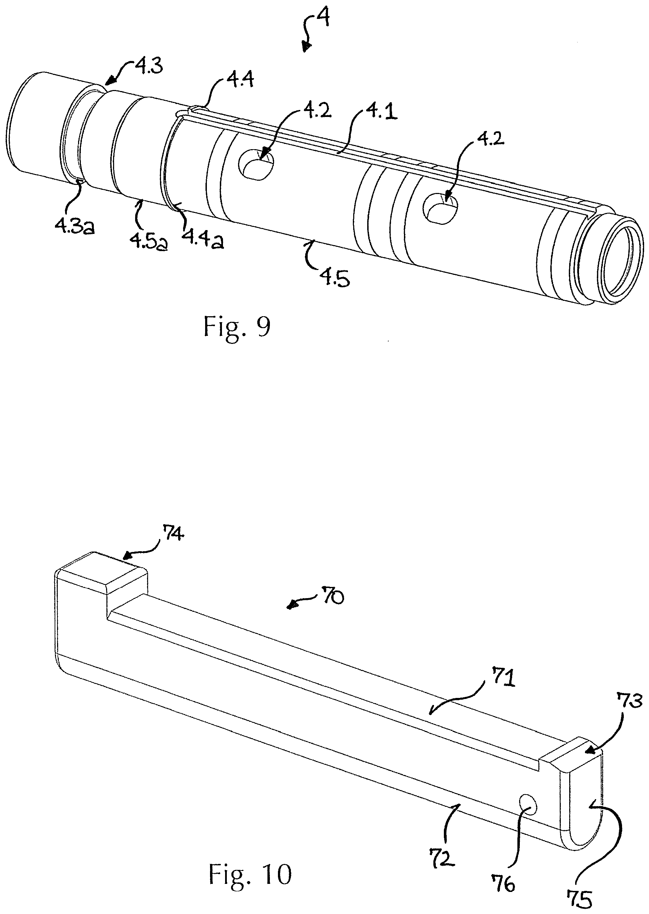

In der Figur 8 ist die Justageeinrichtung 20 in einer Draufsicht von oben gezeigt. Die Justageeinrichtung 20 weist eine ringförmige Auflagefläche 24 auf, welche zumindest teilweise auch durch die Federn 22 gebildet sein kann. Die Auflagefläche 24 ist geometrisch korrespondierend zu einem radial hervorstehenden Rand 4.4 (Figur 9) der Spindel ausgebildet. An einem Außenumfang der Justageeinrichtung 20 sind ferner Befestigungsmittel für eine Verkleidung oder ein Gehäuse vorgesehen. Die Befestigungsmittel sind in Form von Schlitzen 25 vorgesehen, welche sich in radialer Richtung erstrecken. Diese Ausgestaltung erleichtert ein Aufstecken von oben oder von der Seite in radialer Richtung.

In der Figur 9 ist die Spindel 4 im Detail gezeigt. Die Spindel 4 weist zwei in Längsrichtung und gegenüberliegend zueinander angeordnete Axialnuten 4.1 auf. Die jeweilige Axialnut 4.1 erstreckt sich bis zu einem Absatz bzw. radial hervorstehenden Rand 4.4, sodass Federn in die Nuten 4.1 bis hin zum Rand 4.4 geschoben werden können. Die Spindel 4 weist ferner zwei in axialem Abstand zueinander eingebrachte Ausnehmungen 4.2 auf, welche eine Außenmantelfläche 4.5 durchstoßen. Durch die Ausnehmungen 4.2 kann jeweils ein Kabel, insbesondere Schleifringkabel hindurchgeführt werden. Oberhalb des Randes 4.4 ist ein Zentrierflächenabschnitt 4.5a vorgesehen, mittels welchem die Spindel 4 in der Kavität K zentriert werden kann. Durch den Rand 4.4 wird eine ringförmige Auflagefläche 4.4a definiert, an welcher die Justageeinrichtung zur Anlage kommen kann. Durch die umlaufende Nut 4.3 wird eine ringförmige Auflagefläche 4.3a definiert, über welche die Gewichtskraft einer Stativvorrichtung von der Spindel 4 auf die nicht dargestellte Axialsicherung 11 übertragen werden kann.

In der Figur 10 ist ein Sicherungselement 70 in Form eines Riegels im Detail gezeigt. Der Riegel 70 weist eine oberseitige Auflagefläche 71 auf, an welcher die in der Figur 9 gezeigte Auflagefläche 4.3a zur Anlage kommen kann. Ferner weist der Riegel 70 eine unterseitige, im vorliegenden Beispiel gekrümmte, Kontaktfläche 72 auf, mittels welcher der Riegel 70 in der Durchführung 36 an der Montageeinrichtung 30 lagerbar ist. Die Kontaktfläche 72 weist eine Krümmung mit vordefiniertem Krümmungsradius auf, wodurch eine geringe Kerbwirkung sichergestellt werden kann. Die gekrümmte Kontaktfläche 72 kann einen geringen Flächendruck sicherstellen. Der Riegel 70 weist eine U-förmige Querschnittsfläche 75 auf, welche entlang der gesamten Länge des Riegels 70 im Wesentlichen dieselbe Geometrie aufweist. Ein jeweiliges freies Ende des Riegels 70 weist einen Absatz 73, 74 auf, welcher eine Sicherung des Riegels 70 in der Durchführung 36 ermöglicht. Einer der Absätze ist kleiner als der andere.

Der Absatz 73 ist als Montagefase ausgebildet und kann ein Herausrutschen des Riegels 70 verhindern. Der Absatz 74 verhindert ebenfalls ein Herausrutschen. Der Absatz 74 ist bevorzugt derart hoch ausgebildet, dass der Riegel 70 nicht durch die korrespondierende Durchführung geschoben werden kann, sondern am Absatz 74 blockiert.

Im Bereich eines der beiden freien Enden weist der Riegel eine Durchgangsbohrung 76 auf, an welcher der Riegel 70 gesichert werden kann, insbesondere mittels eines Federsteckers.

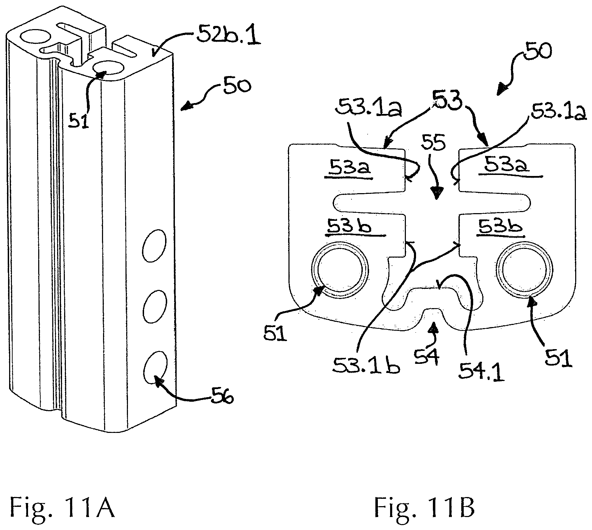

In den Figuren 11A und 11B ist ein Halter 50 gezeigt, welcher mit der in Fig. 6 gezeigten Montageeinrichtung 30 gekuppelt werden kann. Der Halter 50 weist zusätzlich zu den bereits beschriebenen Komponenten Befestigungsmittel 56 auf, welche als quer oder tangential ausgerichtete Öffnungen oder Durchgangsbohrungen ausgebildet sind. Ein jeweiliges Befestigungsmittel, insbesondere die in Fig. 2 gezeigte Schrauben, können durch beide gegenüberliegende Backen 53 gesteckt werden und den Halter 50 an einen entsprechenden Befestigungsabschnitt 33 der Montageeinrichtung 30 anpressen, insbesondere mittels Muttern, so dass von außen eine Druckkraft auf die Mantelflächen der Backen 53 ausgeübt wird. Jede Backe 53 weist am jeweiligen Backenabschnitt Radialflanken 53.1a, 53.1b auf. Die Radialflanken 53.1a, 53.1b sind ebene Flächenabschnitte, welche geometrisch korrespondierend zu den Radialflanken 33.1 der Befestigungsabschnitte 33 ausgebildet sind.

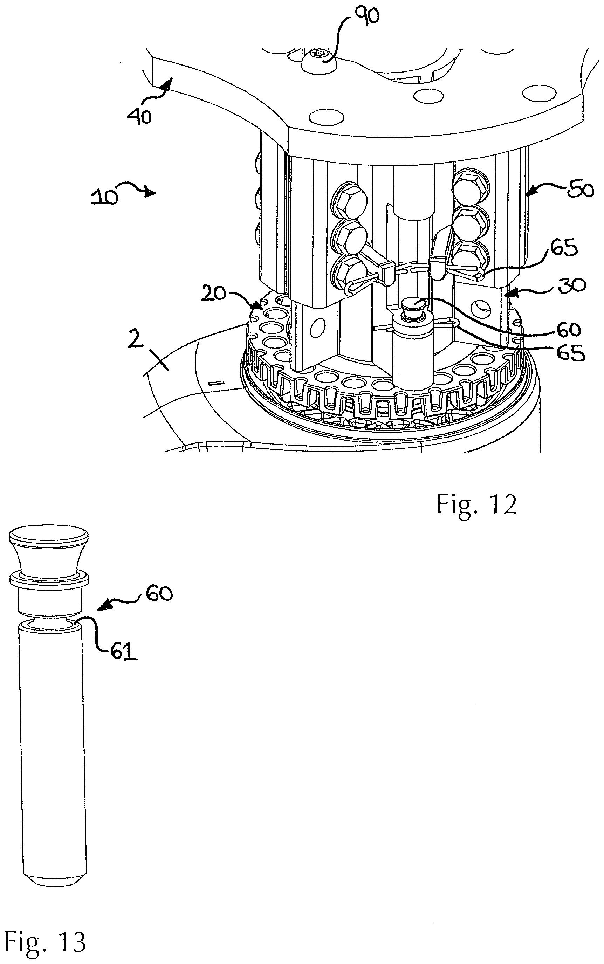

In der Figur 12 ist eine Montagevorrichtung 10 gezeigt, bei welcher ein Verdrehsicherungselement 60 mittels eines Federsteckers 65 an der Montageeinrichtung 30 in Eingriffsposition mit der Justageeinrichtung 20 gesichert ist. Wie aus Figur 13 hervorgeht, greift der Federstecker 65 in eine Nut 61 des Bolzens bzw. Verdrehsicherungselements 60 ein. Die beiden Riegel 70 sind ebenfalls mittels eines entsprechenden Federsteckers 65 gesichert, wobei die Federstecker jeweils in die entsprechende Durchgangsbohrung des jeweiligen Riegels 70 eingreifen.

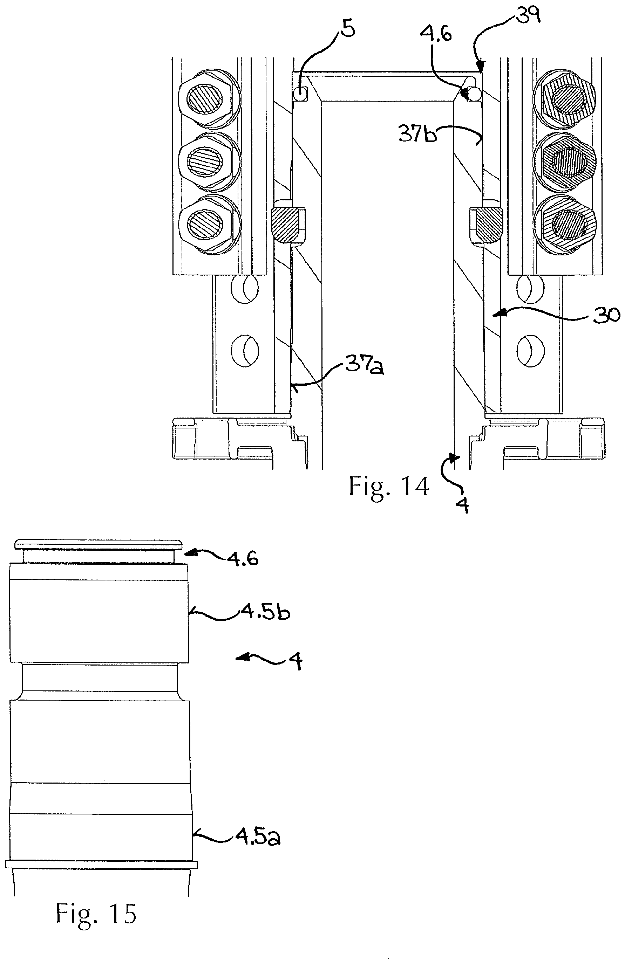

In den Figuren 14 und 15 ist gezeigt, auf welche Weise eine Montageerleichterung mittels zweier Passflächen 37a, 37b und korrespondierender Flächenabschnitte 4.5a, 4.5b an der Spindel erfolgen kann. Die Spindel 4 weist einen Absatz 4.6 auf, an welchem ein O-Ring 5 angeordnet ist. An der Montageeinrichtung 30 ist dazu ein entsprechender Absatz 39 ausgebildet. Die zweite Passfläche 37b weist einen Innendurchmesser auf, welcher kleiner ist als derjenige der ersten Passfläche 37a. Auf diese Weise kann die Spindel 4 zum einen ohne das Risiko eines Verkantens montiert werden, zum anderen kann die Lagerung auf besonders stabile Weise an weit voneinander beabstandeten Flächenabschnitten 4.5a, 4.5b erfolgen.

### Bezugszeichenliste

- 1: Stativvorrichtung, insbesondere Deckenstativvorrichtung
- 2: (erster) Träger oder Tragarm
- 3: zweiter Träger oder Tragarm
- 4: Verbindungsbauteil, insbesondere Spindel
- 4.1: Verdrehsicherung, insbesondere Nut in Verbindungsbauteil, bevorzugt in Längsrichtung eingebrachte Axialnut
- 4.2: Ausnehmung
- 4.3: umlaufende Nut
- 4.3a: ringförmige Auflagefläche
- 4.4: Absatz bzw. radial hervorstehender Rand
- 4.4a: ringförmige Lagerfläche
- 4.5: Außenmantelfläche
- 4.5a: Zentrierflächenabschnitt bzw. erste Passfläche
- 4.5b: zweite Passfläche
- 4.6: Nut für O-Ring am freien Ende der Spindel
- 5: O-Ring

- 10: Montagevorrichtung
- 10a: anpassbarer Mechanismus, insbesondere einstellbare Drehkupplung
- 11: Axialsicherung

- 20: Justageeinrichtung, insbesondere Flachring
- 21: Durchführung
- 22: Verdrehanschlag, insbesondere Feder
- 23: Kupplungspunkt für jeweilige Verdrehposition, insbesondere Loch oder Bohrung
- 24: ringförmige Auflagefläche
- 25: Befestigungsmittel für Verkleidung, insbesondere Schlitz auf Oberseite
- 26: Aussparung, insbesondere an Unterseite, zugänglich von radial außen
- 27: Innenmantelfläche
- 28: Rand bzw. Zentrierung
- 30: Montageeinrichtung, insbesondere als Deckenrohr ausgebildeter Grundkörper, bevorzugt in Form eines Stranggussprofils
- 31: Außenkontur, insbesondere Außenmantelfläche
- 32: Stirnseite
- 32a: untere Stirnseite
- 32a.1: ebene untere Stirnfläche
- 32b: obere Stirnseite
- 32b.1: ebene obere Stirnfläche
- 33: Befestigungsabschnitt, insbesondere Steg
- 33.1: Radialflanke am Befestigungsabschnitt
- 33.1a, 33.1b: gegenüberliegende, insbesondere parallel zueinander angeordnete Radialflanken
- 34: Befestigungsmittel, insbesondere Bohrung oder Gewindebohrung
- 36: Durchführung, insbesondere in tangentialer bzw. radialer Richtung
- 36.1: Auflagefläche
- 37: Innenmantelfläche
- 37a: erste Passfläche
- 37b: zweite Passfläche
- 37.1: Fase, Kante oder Einfräsung
- 38: Aufnahme für Verdrehsicherungselement, insbesondere Sicherungsrohr
- 38a: unterer Aufnahmeabschnitt, insbesondere rohrartig
- 38.1: Durchgangsbohrung, insbesondere in axialer Richtung
- 38.2: Zugang, insbesondere Ausfräsung
- 38.3: oberseitige Zugangsfläche (Schnittstelle)
- 39: Absatz

- 40: Deckenflansch, insbesondere Flanschplatte
- 41: Befestigungsmittel oder Öffnung, insbesondere Innengewindebohrung
- 42a.1: ebene unterseitige Stirnfläche
- 43: Radialnut
- 44: Durchführung
- 44.1: Innenkontur, insbesondere Innenmantelfläche
- 50: Halter, insbesondere in Form eines Stranggussprofils
- 51: Befestigungsmittel, insbesondere axial ausgerichtete Öffnung, bevorzugt Innengewindebohrung
- 52b.1: ebene oberseitige Stirnfläche
- 53: gegenüberliegende Backen
- 53a, 53b: einzelne Backenabschnitte einer Backe
- 53.1a, 53.1b: Führungsfläche oder Radialflanke am jeweiligen Backenabschnitt
- 54: Wellung
- 54.1: Zentrierfläche
- 55: Befestigungsabschnitt, insbesondere Radialkavität
- 56: Befestigungsmittel, insbesondere quer oder tangential ausgerichtete Öffnung, bevorzugt Durchgangsbohrung

- 60: Verdrehsicherungselement, insbesondere Bolzen
- 61: Nut

- 65: Federstecker

- 70: Sicherungselement zur Axialsicherung, insbesondere Riegel
- 71: oberseitige Auflagefläche
- 72: unterseitige Kontaktfläche
- 73: Absatz an einem freien Ende
- 74: Absatz an einem freien Ende
- 75: Querschnittsfläche
- 76: Durchgangsbohrung

- 80: Befestigungselement an Halter, insbesondere Schraube

- 90: Befestigungselement an Flanschplatte, insbesondere Schraube

- D: Montageachse
- G: Gewichtskraft
- H1, H2, H3, ..., Hn: unterschiedliche Höhenpositionen, definiert durch Montagepunkte
- K: Kavität
- x: radiale Richtung bzw. horizontale Richtung
- y: Querrichtung
- z: Längsrichtung bzw. axiale Richtung bzw. vertikale Richtung

## Patentansprüche

1. Montagevorrichtung (10) zur Montage einer Stativvorrichtung (1) unter einer Raumdecke im Operationssaal, umfassend
- eine sich in einer Längsrichtung entlang einer Montageachse (D) erstreckende Montageeinrichtung (30) eingerichtet zum Halten eines Verbindungsbauteils (4) der Stativvorrichtung, wobei die Montageeinrichtung (30) mindestens einen Befestigungsabschnitt (33) mit einer Mehrzahl von Befestigungsmitteln (34) aufweist, welche in Längsrichtung beabstandet zueinander angeordnet sind, und
- einen Deckenflansch (40), eingerichtet zum Montieren der Montageeinrichtung (30) unter der Raumdecke und zum Lagern der Stativvorrichtung an der Raumdecke, wobei der Deckenflansch (40) in unterschiedlichen Montagepositionen entlang der Montageachse (D) relativ zur Montageeinrichtung (30) an der Montageeinrichtung (30) derart montierbar ist, dass die Montagevorrichtung (10) eingerichtet ist, die Stativvorrichtung (1) höhenvariabel in unterschiedlichen, insbesondere vordefinierten Höhenpositionen zu halten,
**dadurch gekennzeichnet, dass**
die Montagevorrichtung (10) mindestens zwei Halter (50) umfasst, mittels derer die Montageeinrichtung (30) in unterschiedlichen Relativpositionen an den Deckenflansch (40) kuppelbar ist, wobei jeder Halter (50) einen Befestigungsabschnitt (53, 56) aufweist, welcher geometrisch korrespondierend zum Befestigungsabschnitt (33) der Montageeinrichtung (30) ausgebildet ist, und
wobei die Montageeinrichtung (30) rohrförmig ausgebildet ist und die Befestigungsabschnitte (33) als radial von ihrer Aussenkontur (31) abragende Stege ausgebildet sind, wobei wenigstens zwei Befestigungsabschitte (33) an der Montageeinrichtung (30) vorgesehen sind, die mit einer korrespondierenden Anzahl an Haltern (50) am Deckenflansch (40) zusammenwirken.

2. Montagevorrichtung (10) nach Anspruch 1, wobei die Befestigungsmittel (34) identisch ausgebildet sind.

3. Montagevorrichtung (10) nach einem der vorhergehenden Ansprüche, wobei die Befestigungsabschnitte (33) sich in Längsrichtung jeweils entlang der gesamten Länge der Montageeinrichtung (30) erstrecken.

4. Montagevorrichtung (10) nach einem der vorhergehenden Ansprüche, wobei der Deckenflansch (40) eine Durchführung (44) aufweist, welche bevorzugt einen Innendurchmesser hat, der größer oder größer gleich ist als ein Außendurchmesser der Montageeinrichtung (30).

5. Montagevorrichtung (10) nach einem der vorhergehenden Ansprüche, wobei der Deckenflansch (40) eine ebene unterseitige Stirnfläche (42a.1) aufweist, an welcher die Montageeinrichtung (30) oder ein Halter (50) zur Anlage bringbar ist, wobei der Deckenflansch (40) bevorzugt als Flanschplatte ausgebildet ist.

6. Montagevorrichtung (10) nach einem der vorhergehenden Ansprüche, wobei der Deckenflansch (40) eine Mehrzahl von Befestigungsmitteln (41), insbesondere Innengewindebohrungen oder Durchgangslöcher aufweist, welche zur Befestigung der Montageeinrichtung (30) am Deckenflansch (40) eingerichtet sind, und welche bevorzugt in Längsrichtung ausgerichtet sind.

7. Montagevorrichtung (10) nach einem der vorhergehenden Ansprüche, wobei der Halter (50) mindestens ein Befestigungsmittel (56) aufweist, welches geometrisch korrespondierend zu Befestigungsmitteln (34) der Montageeinrichtung (30) angeordnet und/oder ausgebildet ist.

8. Montagevorrichtung (10) nach einem der vorhergehenden Ansprüche, wobei der Halter (50) eine Kavität (55) aufweist, insbesondere eine in radialer Richtung ausgerichtete Radialkavität, welche geometrisch derart korrespondierend zum Befestigungsabschnitt (33) der Montageeinrichtung (30) ausgebildet ist, dass der Halter (50) eingerichtet ist, den Befestigungsabschnitt (33) in radialer Richtung zu umgreifen.

9. Montagevorrichtung (10) nach Anspruch 1, wobei die mindestens zwei Befestigungsabschnitte (33) sich in Längsrichtung jeweils entlang der gesamten Länge der Montageeinrichtung (30) erstrecken, wobei der Halter (50) mindestens ein Befestigungsmittel (56) aufweist, welches geometrisch korrespondierend zu Befestigungsmitteln (34) der Montageeinrichtung (30) angeordnet und/oder ausgebildet ist, wobei der Deckenflansch (40) eine unterseitige Stirnfläche (42a.1) aufweist, an welcher der Halter (50) zur Anlage bringbar ist, wobei der Deckenflansch (40) eine Mehrzahl von Befestigungsmitteln (41) aufweist, welche in Längsrichtung ausgerichtet sind und zur Montage des Halters (50) am Deckenflansch (40) eingerichtet sind.

10. Montagevorrichtung (10) nach einem der vorhergehenden Ansprüche, wobei mindestens drei Befestigungsabschitte (33) an der Montageeinrichtung (30) vorgesehen sind, die mit einer korrespondierenden Anzahl an Haltern (50) am Deckenflansch (40) zusammenwirken.

11. Montagevorrichtung (10) nach einem der vorhergehenden Ansprüche, wobei das Verbindungsbauteil (4) eine Spindel ist.

12. Montagesystem mit einer Montagevorrichtung (10) nach einem der vorhergehenden Ansprüche, wobei das Montagesystem ferner umfasst:
- mindestens ein Befestigungselement (90) zur Montage des Deckenflansches (40) an der Montageeinrichtung (30) oder an einem mit der Montageeinrichtung (30) verbundenen Halter (50), und/oder
- mindestens ein Befestigungselement (80) zur Montage mindestens eines Halters (50) an der Montageeinrichtung (30).

13. Montagesystem nach Anspruch 12, wobei das Montagesystem ferner umfasst:
- eine Justageeinrichtung (20) zur Anordnung eines Verbindungsbauteils der Stativvorrichtung (1) in einer vordefinierbaren Position relativ zur Montageeinrichtung (30), wobei die Montagevorrichtung eine um die Montageachse einstellbare Drehkupplung zum Lagern des Verbindungsbauteils an der Montageeinrichtung bildet.

14. Montagesystem nach Anspruch 12 oder 13, wobei das Montagesystem ferner umfasst:
- ein erstes Element zur Verdrehsicherung (60), insbesondere einen Bolzen, und
- ein zweites Element zur Axialsicherung (70), insbesondere einen Riegel.

15. Montagesystem nach einem der Ansprüche 12 bis 14, wobei das Befestigungselement (90) zur Montage des Deckenflansches (40) und/oder das Befestigungselement (80) zur Montage mindestens eines Halters (50) eine Schraube ist.

16. Stativvorichtung (1), umfassend
- ein Montagesystem nach einem der Ansprüche 12 bis 15, und
- das Verbindungsbauteil (4), wobei das Verbindungsbauteil bevorzugt eine Spindel ist, welche/s axialfest an der Montageeinrichtung (30) der Montagevorrichtung (10) des Montagesystems gelagert oder befestigt ist.

## Claims

1. A mounting device (10) for mounting a stand device (1) under a ceiling in an operating room, comprising
- a mounting apparatus (30) extending in a longitudinal direction along a mounting axis (D) configured to hold a connection component (4) of the stand device, wherein the mounting apparatus (30) comprises at least one fastening portion (33) including a plurality of fastening means (34) which are spaced to one another in the longitudinal direction, and
- a ceiling flange (40) configured to mount the mounting apparatus (30) under the ceiling and to support the stand device on the ceiling; wherein the ceiling flange (40) is mountable in different mounting positions along the mounting axis (D) relative to the mounting apparatus (30) on the mounting apparatus (30) in a way that the mounting device (10) is configured to hold the stand device (1) in a height variable manner in different, in particular predefined, height positions,
**characterized in that**
the mounting device (10) comprises at least two supports (50) by means of which the mounting apparatus (30) may be coupled to the ceiling flange (40) in different relative positions, wherein each support (50) comprises a fastening portion (53, 56) which is formed to correspond geometrically to a fasting portion (33) of the mounting apparatus (30), and
wherein the mounting apparatus (30) is adapted as tube and the fastening portions (33) are adapted as crosspieces protruding radially from the outer contour (31), wherein at least two fastening portions (33) are provided on the mounting apparatus (30) which work together with a corresponding number of supports (50) on the ceiling flange (40).

2. The mounting device (10) according to claim 1, wherein the fastening means (34) are formed identically.

3. The mounting device (10) according to any one of the preceding claims, wherein the fastening portions (33) extend along the complete length of the mounting apparatus (30) in the longitudinal direction, respectively.

4. The mounting device (10) according to anyone of the preceding claims, wherein the ceiling flange (40) comprises a passage (44), which preferably has an inner diameter that is larger or larger equal than an outer diameter of the mounting apparatus (30).

5. The mounting device (10) according to one of the preceding claims, wherein the ceiling flange (40) comprises a planar lower side front face (42a.1) where the mounting apparatus (30) or a support (50) can abut, wherein the ceiling flange (40) is preferably formed as a flange plate.

6. The mounting device (10) according to any one of the preceding claims, wherein the ceiling flange (40) comprises a plurality of fastening means (41), in particular internally threaded bores or through-holes, which are configured to fasten the mounting apparatus (30) on the ceiling flange (40), and which are preferably aligned in the longitudinal direction.

7. The mounting device (10) according to any one of the preceding claims, wherein the support (50) includes at least one fastening means (56) which is arranged and/or formed to correspond geometrically to fastening means (34) of the mounting apparatus (30).

8. The mounting device (10) according to any one of the preceding claims, wherein the support (50) includes a cavity (55), in particular a radial cavity aligned in a radial direction which is formed to correspond geometrically to the fastening portion (33) of the mounting apparatus (30) in a way that the support (50) is configured to enclose the fastening portion (33) in the radial direction.

9. The mounting device (10) according to claim 1, wherein the at least two fastening portions (33) extend along the complete length of the mounting apparatus (30) in the longitudinal direction, respectively, wherein the support (50) comprises at least one fastening means (56) which is arranged and/or formed to correspond geometrically to fastening means (34) of the mounting apparatus (30), wherein the ceiling flange (40) comprises a lower front face (42a.1) where the support (50) can abut, wherein the ceiling flange (40) comprises a plurality of fastening means (41) that are aligned in the longitudinal direction and are configured to mount the support (50) on the ceiling flange (40).

10. The mounting device (10) according to any one of the preceding claims, wherein at least three fastening portions (33) are provided on the mounting apparatus (30) which work together with a corresponding number of supports (50) on the ceiling flange (40).

11. The mounting device (10) according to any one of the preceding claims, wherein the connection component (4) is a spindle.

12. A mounting system comprising a mounting device (10) according to any one of the preceding claims, wherein the mounting system further comprises:
- at least one fastening element (90) for mounting the ceiling flange (40) on the mounting apparatus (30) or on a support (50) coupled with the mounting apparatus (30), and/or
- at least one fastening element (80) for mounting the at least one support (50) on the mounting apparatus (30).

13. The mounting system according to claim 12, wherein the mounting system further comprises:
- an adjustment apparatus (20) to arrange a connection component of the stand device (1) in a predefinable position relative to the mounting apparatus (30), wherein the mounting device forms a rotational coupling adjustable around the mounting axis to support the connection component on the mounting apparatus.

14. Mounting system according to claim 12 or 13, wherein the mounting system further comprises:
- a first element for rotational lock (60), in particular a bolt and
- a second element for axial lock (70), in particular a latch.

15. Mounting system according to any one of claims 12 to 14, wherein the fastening element (90) for mounting the ceiling flange (40) and/or the fastening element (80) for mounting the at least one support (50) is a screw.

16. Stand device (1), comprising
- a mounting system according to any one of claims 12 to 15, and
- a connection component (4), wherein the connection component is preferably a spindle, which is supported or fastened in an axially secured manner on the mounting apparatus (30) of the mounting device (10) of the mounting system.

## Revendications

1. Dispositif de montage (10) prévu au montage d'un dispositif de trépied (1) sous un plafond dans la salle d'opération, comportant
- un organe de montage (30) s'étendant en direction longitudinale selon un axe de montage (D), conçu pour soutenir un élément de connexion (4) du dispositif de trépied, l'organe de montage (30) comportant au moins un tronçon de fixation (33) comprenant une pluralité de moyens de fixation (34) espacés longitudinalement l'un de l'autre, et
- une bride de plafond (40) prévue au montage de l'organe de montage (30) sous un plafond et au stockage du dispositif de trépied au plafond, la bride de plafond (40) pouvant être montée en différentes positions de montage le long de l'axe de montage (D) relativement par rapport à l'organe de montage (30) de manière que le dispositif de montage (10) est apte à maintenir le dispositif de trépied (1) variablement en hauteur en différentes positions de hauteur notamment prédéfinies,
**caractérisé en ce que**
le dispositif de montage (10) comporte au moins deux attaches (50) permettant le couplage de l'organe de montage (30) à la bride de plafond (40) en différentes positions relatives, l'attache (50) comportant un tronçon de fixation (53, 56) configuré de façon géométriquement et correspondante par rapport au tronçon de fixation (33) de l'organe de montage (30), et
la forme de l'organe de montage (30) est tubulaire, les tronçons de fixation (33) étant conçus sous forme d'ailettes s'étendant radialement depuis leur contour extérieur (31), deux tronçons de fixation (33) au moins étant prévus sur l'organe de montage (30), ceux-ci coopérant avec un nombre correspondant d'attaches (50) sur la bride de plafond (40).

2. Dispositif de montage (10) selon la revendication 1, dans lequel les moyens de fixation (34) sont de configuration identique.

3. Dispositif de montage (10) selon l'une des revendications précédentes, dans lequel les tronçons de fixation (33) s'étendent chacun selon la longueur totale de l'organe de montage (30).

4. Dispositif de montage (10) selon l'une des revendications précédentes, dans lequel la bride de plafond (40) présente un passage (44) ayant de préférence un diamètre intérieur égal ou supérieur à un diamètre extérieur de l'organe de montage (30).

5. Dispositif de montage (10) selon l'une des revendications précédentes, dans lequel la bride de plafond (40) présente une surface inférieure (42a.1) permettant le serrage de l'organe de montage (30) ou d'une attache (50), la bride de plafond (40) étant configurée de préférence comme plaque formant bride.

6. Dispositif de montage (10) selon l'une des revendications précédentes, dans lequel la bride de plafond (40) comporte une pluralité de moyens de fixation (41), notamment des alésages à filetage femelle ou des trous traversants, conçus pour la fixation de l'organe de montage (30) à la bride de plafond (40) et s'étendant de préférence en direction longitudinale.

7. Dispositif de montage (10) selon l'une des revendications précédentes, dans lequel l'attache (50) comporte au moins un moyen de fixation (56) disposé et / ou configuré de manière géométriquement correspondante à des moyens de fixation (34) de l'organe de montage (30).

8. Dispositif de montage (10) selon l'une des revendications précédentes, dans lequel l'attache (50) présente une cavité (55), notamment une cavité radiale orientée dans le sens radial, dont la forme correspond géométriquement de telle manière au tronçon de fixation (33) de l'organe de montage (30) que l'attache (50) puisse enserrer le tronçon de fixation (33) dans le sens radial.

9. Dispositif de montage (10) selon la revendication 1, dans lequel les au moins deux tronçons de fixation (33) s'étendent longitudinalement chacun selon la longueur totale de l'organe de montage (30), l'attache (50) comportant au moins un élément de fixation (56) disposé et / ou de manière géométriquement correspondante par rapport aux moyens de fixation (34) de l'organe de montage (30), la bride de plafond (40) ayant une surface inférieure (42a.1) permettant le serrage de l'attache (50), la bride de plafond (40) comportant une pluralité de moyens de fixation (41) orientés longitudinalement et permettant le montage de l'attache (50) sur la bride de plafond (40).

10. Dispositif de montage (10) selon l'une des revendications précédentes, dans lequel au moins trois tronçons de fixation (33) sont prévus sur l'organe de montage (30) qui coopèrent avec un nombre correspondant d'attaches (50) sur la bride de plafond (40).

11. Dispositif de montage (10) selon l'une des revendications précédentes, dans lequel l'élément de connexion (4) est une goupille.

12. Système de montage comportant un dispositif de montage (10) selon l'une des revendications précédentes, dans lequel le système de montage comporte par ailleurs :
- au moins un élément de fixation (90) servant au montage de la bride de plafond (40) sur l'organe de montage (30) ou sur une attache (50) reliée à l'organe de montage (30), et / ou
- au moins un élément de fixation (80) prévu au montage d'au moins une attache (50) sur l'organe de montage (30).

13. Système de montage selon la revendication 12, dans lequel le système de montage comporte par ailleurs :
un dispositif d'ajustage (20) pour placer un élément de connexion du dispositif de trépied (1) dans une position prédéfinissable par rapport à l'organe de montage (30), le dispositif de montage formant un accouplement rotatif ajustable autour de l'axe de montage pour le stockage de l'élément de connexion sur l'organe de montage.

14. Système de montage selon la revendication 12 ou 13, dans lequel le système de montage comporte par ailleurs :
- un premier dispositif anti-rotation (60), notamment un boulon, et
- un deuxième dispositif de blocage axial (70), notamment un verrou.

15. Système de montage selon l'une des revendications 12 à 14, dans lequel, quant à l'élément de fixation (90) servant au montage de la bride de plafond (40) et / ou l'élément de fixation (80) servant au montage d'au moins une attache (50), il s'agit d'une vis.

16. Dispositif de trépied (1), comportant :
- un système de montage selon l'une des revendications 12 à 15, et
- l'élément de connexion (4), l'élément de connexion étant de préférence une goupille montée ou fixée axialement fixe sur l'organe de montage (30) du dispositif de montage (10) du système de montage.
